# EUROPEAN PATENT SPECIFICATION

(11) **EP 4 402 633 B1**
(45) Date of publication and mention of the grant of the patent: **28.10.2026**
(21) Application number: 22868486.6
(22) Date of filing: 20.05.2022
(51) Int. Cl.: G06T 1/00, A61C 9/00, A61C 13/00, G06F 16/901, G06T 9/00, G06F 30/00, G06T 15/06

(54) **SYSTEM AND METHOD FOR AUGMENTED INTELLIGENCE IN DENTAL FILE SEGMENTATION**
SYSTEM UND VERFAHREN FÜR ERWEITERTE INTELLIGENZ BEI DER SEGMENTIERUNG ZAHNÄRZTLICHER DATEIEN
SYSTÈME ET PROCÉDÉ POUR UNE INTELLIGENCE AMPLIFIÉE DANS UNE SEGMENTATION DE FICHIER DENTAIRE

(30) Priority: 14.09.2021 US 202163243866 P
(43) Date of publication of application: 24.07.2024
(73) Proprietor: Aicad Dental Inc., Chelsea, QC J9B 1P4 (CA)
(72) Inventor: COULOMBE, Fabien, Chelsea, Québec J9B 1P4 (CA); PATEL, Akshaykumar Ketankumar, Ottawa, Ontario K1G 3P7 (CA)
(74) Representative: Bosch Jehle Patentanwaltsgesellschaft mbH
(86) International application number: PCT/CA2022/050814
(87) International publication number: WO 2023/039658

(56) References cited:
- US-A1- 2017 100 220
- US-A1- 2017 262 978
- US-B1- 8 013 853
- "Computer Vision Metrics: Survey, Taxonomy, and Analysis", 22 May 2014, APRESS BERKELEY, CA, USA, article KRIG, S.: "Passages; Computer Vision Metrics: Survey, Taxonomy, and Analysis", pages: 154, 204,215,270, XP009544655, DOI: 10.1007/978-1-4302-5930-5
- ZONGYI LI; NIKOLA KOVACHKI; KAMYAR AZIZZADENESHELI; BURIGEDE LIU; KAUSHIK BHATTACHARYA; ANDREW STUART; ANIMA ANANDKUMAR: "Fourier Neural Operator for Parametric Partial Differential Equations", ARXIV.ORG, CORNELL UNIVERSITY LIBRARY, 201 OLIN LIBRARY CORNELL UNIVERSITY ITHACA, NY 14853, 17 May 2021 (2021-05-17), 201 Olin Library Cornell University Ithaca, NY 14853 , XP081951358

## Description

### CROSS-REFERENCE TO RELATED APPLICATION

This application claims priority to United States provisional patent application 63/243,866 filed 14 September 2021

### FIELD OF THE INVENTION

The present invention pertains to the field of dental prosthetic and implant design. The present invention also pertains to dental image file capture and manipulation for the purpose of dental tracking, and dental prosthetic and implant design.

### BACKGROUND OF THE INVENTION

Dental imaging technologies are used in a modern dental practice to image the mouth and teeth, as well as the underlying bone and jaw structure. Dental imaging can provide a wide range of information for improving the care of patients, including for monitoring tooth movement, gum changes, for designing dental implants and prosthetics, and for investigations prior to surgery. In orthodontics dental imaging is used to plan treatments for alignment of teeth, including designing and creating dental appliances, such as orthodontic aligners, to align the patient's teeth according to the treatment plan.

In the design of dental prosthetics and implants, for example, there are a variety of imaging technologies and associated dental computer aided design (CAD) software technologies are used to provide 2D as well as 3D images of the teeth, gums, and mouth to enable a technician to design and build an implant that fits the patient. Imaging is generally done using one or more optical cameras, or using x-rays such as with computed tomography (CT) or radiography. By combining multiple 2D images or cross sectional images a 3D image of the mouth or teeth area can be constructed as a mosaic of 2D images. A dental implant or prosthetic can be designed based on this captured 3D image of the mouth.

In an example, United States patent US10,426,578B2 to Rubbert et al. describes a dental implant system for a dental prosthesis/implant which includes a dental implant body having a prosthesis interface formed to receive an occlusal-facing dental prosthesis component. The prosthesis interface has a custom three-dimensional surface shape positioned and formed to create a form locking fit with respect to the occlusal-facing dental prosthesis component when positioned thereon.

A 3D image captured of the teeth and gums can be further converted by shaping 3D images in various tessellated data points format, also referred to as meshing, to create a stereolithography or "STL" format of the 3D image, and these mesh files can be used in orthotic design. In one example, United States patent US11,191,618B1 to Raslambekov describes a method and a system for manufacturing an orthodontic appliance by receiving a 3D mesh including a plurality of inner vertices representative of an inner surface of an appliance, generating a plurality of outer vertices representative of an outer surface of the appliance, and causing the manufacturing of the appliance based on the appliance 3D representation.

Processing meshed data is highly processing and computer central processing unit (CPU) intensive and using native file formats which are very large increases the time and cost of image processing. In the current digital workflows of dental prosthetic manufacture, computer aided design (CAD) of dental appliances and implants is, by far, the most labor-intensive step in the dental prosthetic manufacturing process. Computer-Aided Manufacturing (CAM) of dental frameworks using 3D printers and computerized numerical control (CNC) is comparably much faster than CAD, requiring little skill labor and the material required for manufacture is quickly getting cheaper. Understanding that CAD is a bottleneck for current dental CAD-CAM workflows, there remains a need for more accurate and efficient site preparation, imaging, and image processing at the dental office with the patient prior to manufacturing as a crucial step of design of a dental crown, implant, or appliance.

United States patent application US2017/262978A1 to Reynolds describes a medical image data processing system whereby a three-dimensional medical imaging data set is processed to determine curved planes in the image data set. United States patent application US2017/100220A1 to Kopelman and Taub describes a method for manipulating a dental virtual model to create trimmed virtual models and trimmed physical models which can be used in the design and manufacture of copings or prostheses.

This background information is provided for the purpose of making known information believed by the applicant to be of possible relevance to the present invention.

### SUMMARY OF THE INVENTION

It is an object of the present invention to provide a system and method for dental image file capture and manipulation for the purpose of dental tracking, and dental prosthetic and implant design. It is another object of the present invention to provide a system and method for augmented intelligence in dental file segmentation for expediting the design and manufacture of dental prosthetics, appliances, and in dental monitoring and treatment.

Said method and system are defined in claim 1 and 12 respectively.

In an aspect of the present invention there is provided a computer-implemented method for dental file segmentation comprising: receiving a 3D mesh file representation of a 3D dental object; aligning a longitudinal axis of the 3D mesh file with a z-axis; assigning a slicing centroid to the 3D mesh file; slicing the 3D mesh file into an plurality of cross-sectional slices; and for each 2D cross-sectional slice: identifying an indexing centroid; measuring a length of each of a plurality of indexing rays to provide a plurality of lengths, each of the plurality of indexing rays extending from the indexing centroid to a cross-sectional boundary of the cross-sectional slice; and storing the plurality of lengths in a row in a 2D matrix to create a 2D descriptor matrix of the 3D mesh file

In an embodiment, the method further comprises classifying the dental object with a dental descriptor.

In another embodiment, the dental descriptor comprises one or more of dental object location, tooth identification, quadrant location, dental arch, bitewing, occlusal surface, and gumline.

In another embodiment, each of the plurality of cross-sectional slices is related at a selected alpha angle.

In another embodiment, the plurality of indexing rays are separated by a selected beta angle.

In another embodiment, the method further comprises matching the 2D matrix to one or more similar 2D matrix in a descriptor database.

In an embodiment, the method further comprises using the selected one or more similar 2D matrix to design a dental prosthetic.

In an embodiment, the method further comprises manufacturing the dental prosthetic.

In an embodiment, the method further comprises rendering the 2D matrix as a visualization map image.

In another embodiment, the visualization map is rendered such that each entry in the descriptor matrix is replaced with a corresponding color.

In another embodiment, slicing the 3D mesh file comprises radially slicing the dental object through the slicing centroid.

In another embodiment, slicing the 3D mesh file comprises longitudinally slicing the dental object into substantially parallel slices.

In another embodiment, the dental object is one or more of a single tooth, multiple teeth, two or more adjacent teeth, full dental arch, half dental arch, dental quadrant, bitewing, and gumline.

In another aspect of the present invention there is provided computer implemented system for dental file segmentation comprising: a dental object descriptor generating system comprising: an indexed slicer for receiving a 3D mesh file of a dental object and slice the mesh file into a plurality of slices, each slice comprising a cross-sectional boundary of the dental object; a radial encoder assigning an indexing centroid and measuring a plurality of rays from the indexing centroid to the cross-sectional boundary; a Fourier neural operator unit to transform the plurality of ray lengths into a 2D descriptor matrix of the dental object; and a descriptor database comprising a plurality of 2D descriptor matrixes for a plurality of dental objects, each 2D matrix having a descriptor classification.

In an embodiment, the system further comprises a cluster model for matching two or more of the plurality of 2D descriptor matrixes in the descriptor database.

In another embodiment, the system further comprises a computer-aided design system for designing a dental prosthetic based on the 2D descriptor matrix.

In another embodiment, the system further comprises a dental scanner.

In another aspect of the present invention there is provided a system for dental file segmentation comprising: at least one processor; and a memory storing instructions which when executed by the at least one processor configure the at least one processor to: receive a 3D mesh file representation of a 3D dental object; align a longitudinal axis of the 3D mesh file with a z-axis; assign a slicing centroid to the 3D mesh file; slice the 3D mesh file into an plurality of cross-sectional slices; and for each 2D cross-sectional slice: identify an indexing centroid; measure a length of each of a plurality of indexing rays, each indexing ray extending from the indexing centroid to a cross-sectional boundary of the slice; and store the length of each of a plurality of indexing rays in a row in a 2D matrix to create a 2D descriptor matrix of the 3D mesh file.

In an embodiment, the system further comprises a descriptor database comprising a plurality of 2D descriptor matrixes for a plurality of dental objects.

In another embodiment, storing the plurality of indexing ray lengths in a row in a 2D matrix comprises applying a Fourier neural operator to the plurality of indexing rays to transform the plurality of ray lengths by normalizing the length of each ray distance to the indexing centroid.

In accordance with another aspect, there is provided a computer-implemented system for generating two-dimensional (2D) descriptors for three-dimensional (3D) objects comprising at least one processor and a memory storing instructions which when executed by the at least one processor configure the at least one processor to slice a three-dimension (3D) representation of an object into an equal number of two-dimension (2D) cross-section slices, for each 2D cross-section slice determine a centroid and a plurality of radial lengths, store the plurality of radial lengths in a first descriptor matrix, and render the first descriptor matrix such that each entry in the first descriptor matrix is replaced with a corresponding color. Each radial length is between the centroid and a different point on a perimeter of the cross-section. Each radial length is separated by a same angle measured from the centroid. A first dimension of the first descriptor matrix comprises a number of the plurality of cross-section slices. A second dimension of the first descriptor matrix comprises a number of the plurality of radial lengths in each slice.

In accordance with another aspect, there is provided a computer-implemented method of generating two-dimensional (2D) descriptors for three-dimensional (3D) objects are provided. The method comprises slicing a three-dimension (3D) representation of an object into an equal number of two-dimension (2D) cross-section slices, for each 2D cross-section slice determining a centroid and a plurality of radial lengths, storing the plurality of radial lengths in a first descriptor matrix, and rendering the first descriptor matrix such that each entry in the first descriptor matrix is replaced with a corresponding color. Each radial length is between the centroid and a different point on a perimeter of the cross-section. Each radial length is separated by a same angle measured from the centroid. A first dimension of the first descriptor matrix comprises a number of the plurality of cross-section slices. A second dimension of the first descriptor matrix comprises a number of the plurality of radial lengths in each slice.

In various further aspects, the disclosure provides corresponding systems and devices, and logic structures such as machine-executable coded instruction sets for implementing such systems, devices, and methods.

In this respect, before explaining at least one embodiment in detail, it is to be understood that the embodiments are not limited in application to the details of construction and to the arrangements of the components set forth in the following description or illustrated in the drawings. Also, it is to be understood that the phraseology and terminology employed herein are for the purpose of description and should not be regarded as limiting.

### BRIEF DESCRIPTION OF THE FIGURES

Embodiments will be described, by way of example only, with reference to the attached figures, wherein in the figures:
FIG. 1 illustrates, in a component diagram, an example of a dental file segmentation system, in accordance with some embodiments.
FIG. 2 illustrates, in a schematic diagram, an example of a descriptor generation system , in accordance with some embodiments.
FIG. 3A illustrate examples outputs of components of the descriptor generation system employing an example of a single tooth description method, in accordance with some embodiments.
FIG. 3B illustrate an example of a dental object analyzed by an indexed slicer sliced radially into a plurality of radial portions.
FIG. 3C illustrates an example cross-sectional plane from the indexed slicer sliced into indexing rays.
FIG. 3D illustrates an example of a radial encoder output as a 2D matrix.
FIG. 3E is an illustration of a visualization map of the radial encoder output as a pixel array.
FIG. 4 is a flow chart illustrating an example computational method of generating a single tooth descriptor.
FIG. 5 is an example method of dental file segmentation and creation of a 2D representation for a dental object.
FIG. 6A illustrate examples outputs of components of the descriptor generation system employing an example of another single tooth description method, in accordance with some embodiments.
FIG. 6B illustrates an example of a portion of the three-dimensional quadrant with longitudinal slicing planes.
FIG. 6C illustrates an example of a radial encoding of a tooth through a slicing plane.
FIG. 6D illustrates another example of the output 2D matrix of the Fourier neural operator for the plane of a tooth.
FIG. 6E illustrates an example of a visualization mapping/image using a black and white greyscale mapping.
FIG. 7 illustrates, in a flow chart, another example of a method of generating a single tooth descriptor, in accordance with some embodiments.
FIG. 8A illustrates alignment of a dental object with a single tooth and adjacent teeth.
FIG. 8B illustrates the indexing of one cross section plane of a tooth.
FIG. 8C is an image of a tooth which is processed by an indexing centroid positioned at a relatively infinite distance from the tooth.
FIG. 9A illustrates a dental quadrant as the dental object in a mesh or STL file format
FIG. 9B illustrates an example slicing of a dental object in parallel longitudinal slicing planes.
FIG. 9C shows a single parallel slicing plane and dental object cross sectional boundary.
FIG. 9D shows an example of an output 2D matrix of the Fourier neural operator for a dental object.
FIG. 9E illustrates an example of a matrix visualization using greyscale mapping.
FIG. 10 illustrates, in a flowchart, an example of a method of generating a dental arch descriptor, in accordance with some embodiments.
FIG. 11 is an example system flow diagram for designing a dental prosthetic.
FIG. 12A illustrates an example of a dental quadrant in a mesh file format.
FIG. 12B illustrates a dental quadrant which has been passed through an indexed slicer.
FIG. 12C shows a single slicing plane and dental object cross sectional boundary.
FIG. 12D shows an example of an output 2D matrix of the Fourier neural operator for a dental object.
FIG. 12E illustrates an example of a matrix visualization using greyscale mapping.
FIG. 13 illustrates, in a flowchart, another example of a method of generating a dental arch descriptor, in accordance with some embodiments.
FIG. 14A illustrates an example of the z-axis position for a quadrant in a dental arch.
FIG. 14B illustrate planar slices of a dental arch.
FIG. 14C shows a single slicing plane and dental object cross sectional boundary.
FIG. 15 illustrates, in a flowchart, an example of a method of generating a full arch descriptor, in accordance with some embodiments.
FIG. 16A illustrates an example of a full arch, in accordance with some embodiments.
FIG. 16B illustrates an example of half of the full arch after splitting the full quadrant, in accordance with some embodiments.
FIG. 16C illustrates an example of another half of the full arch after splitting the full quadrant.
FIG. 17 illustrates dental descriptors for single and multiple dental features.
FIG. 18A illustrates a training model workflow for a class one single tooth descriptor classification.
FIG. 18B illustrates an example workflow for crown production using a single tooth descriptor classification.
FIG. 19A illustrates an array mapping procedure for crown post fitting.
FIG. 19B illustrates a crown environment fitting with a fitted crown.
FIG. 19C illustrates a piece of an array mapping procedure for crown fitting.
FIG. 20A is an illustration of a crown mounted onto a post showing a cross section of the abutment surface.
FIG. 20B is an illustration of a crown mounted onto a post showing a cross section of the abutment surface between the outside of the crown and the distal and mesial abutments to neighbouring teeth.
FIG. 20C is a visual representation of a combination of feature matrixes to provide a fulsome value mapping of a crown.
FIG. 20D illustrates an example of a matrix stacking of a plurality of 2D matrixes, with each matrix providing a value mapping of a different feature of the crown in its dental environment.
FIG. 21 illustrates an array value mapping method for a dental arch.

### DETAILED DESCRIPTION

Embodiments of methods, systems, and apparatus are described through reference to the drawings. Applicant notes that the described embodiments and examples are illustrative and nonlimiting. Practical implementation of the features may incorporate a combination of some or all of the aspects, and features described herein should not be taken as indications of future or existing product plans.

Herein is provided a system and method for dental image file capture and manipulation for the purpose of dental tracking, and dental prosthetic and implant design. It is another object of the present invention to provide a system and method for augmented intelligence in dental file segmentation for expediting the design and manufacture of bespoke dental prosthetics, appliances, crowns, and in dental monitoring and treatment using a methodology involving the processing of tessellated file formats. This presently described system and method provides an expedited method of manufacture of dental appliances using less data which uses less processing time. In addition, the present system and method can be used to reduce design and manufacturing time as well as processing energy required, particularly in the CAD/CAM industry. Other industries that use tessellated surfaces in their workflow may also benefit from the present method which reduces file size and increases processing speed of file comparison and utilization in artificial intelligence matching algorithms.

In the present system and method, dental objects are imaged and stored as three dimensional (3D) STL files, whose name is derived from "Standard Tessellation Language", also referred to herein as mesh files. An STL file describes a raw, unstructured triangulated surface using unit normal and vertices of the triangles using a three-dimensional Cartesian coordinate system. The STL or mesh file can be understood as a 3D tessellated image representation of an object, where the triangles in the mesh file form the 3D tessellation describing the object in 3D cartesian space. The term "tessellation" as used herein refers to the covering of a surface, often a plane, using a plurality of triangles, with no overlaps and no gaps. The STL or mesh file is converted into a set of two dimensional (2D) arrays or matrices using a digital encoder, where each array describes one or more key feature of one or more tooth or dental structure. In practice, 3D STL files are segmented into a set of two-dimensional (2D) arrays, also referred to herein as matrices, where each array describes one or more keys feature of a complex object, such as a dental object. The small and highly compact data set provided by the set of matrices represent more than one key feature of a patient's mouth and dentition and can be used in time lapse dental monitoring and care, as well as in the manufacturing of unique and bespoke dental appliances and prosthetics with efficiency of data matching, comparison, and processing.

The present disclosure may be described herein in terms of various components and processing steps in a method for augmented intelligence in dental file segmentation and efficient dental image file capture and manipulation for the purpose of dental tracking, and dental prosthetic and implant design. Each component provides different features of a digital description and representation of dental anatomy. Some processing steps described herein include an indexed slicer, a radial encoder, a Fourier neural operator, and a visualization device or graphical user interface for viewing the image file. The image output provided to the graphical user interface may, for example, be stacked to facilitate all the features into one object. In some embodiments, an indexed slicer divides dental anatomy into slices so that a cross-section can be created which can further be used in a radial encoder. In some embodiments, the radial encoder maps the cross-section from a particular slicing centroid. Once a mapping has been completed the next step is to store the data strategically. In some embodiments, this is achieved by a Fourier neural operator and an output visual rendering for each 2D array or matrix value mapping or visualisation that allows a field expert such as, for example, a dentist, dental technician, data scientist, or software developer, to recognize each key dental feature described by each array.

Two or more 2D matrixes may also be stacked to describe the full dental anatomy. In an example, the matrices for a dental patient can be categorized into a tooth descriptor stack, a quadrant descriptor stack, a dental arch descriptor stack, and/or a shade pattern descriptor stack. Furthermore, a bite pattern and a bite registration descriptor for a dental patient may be provided in a similar manner as the encoding method. These descriptors allow for at least two output formats: a 2D matrix array or matrix file format; and an image format. With these descriptors, a dental professional can review, for example, the bite, occlusion surfaces, gum shape, dental arch, and tooth features, to inform the design of tailored dental appliances, crowns, dental prosthetics such as bridges, and to monitor patient dental health. In addition, the dental professional can visualize and monitor the patient's mouth and teeth to compare before and after a dental implant or crown.

The teachings herein may be implemented in any number of dental contexts and for a multitude of dental uses. These include, for example, dental tracking, monitoring tooth movement, gum changes, dental prosthetic and implant design, and for investigations prior to and after surgery, anomaly detection, pattern recognition, and similar dental anatomy search. Initial dental and dentition images may be captured in many different ways, for example using any type of scanner, including but not limited to a laser scanner, optical scanner, ultrasound, camera, or x-ray. The scanned image can then be used to digitize the dental object or dental image in its conversion to a mesh file or STL file for use in the present method and system. A multitude of systems, devices, and methods are known in the dental industry for dental scanning, and there are a variety of methods for converting the scanned image file into a mesh file. One skilled in the art understands that the methods described below may import mesh files or STL files from various formats including binary/ASCII STL, Wavefront OBJ, ASCII OFF, binary/ASCII PLY, GLTF/GLB 2.0, 3MF, XAML, 3DXML, etc. For simplicity of the present description, the STL format will be referred to herein as a mesh file. The methods described herein are a few of the exemplary applications for the present disclosure. The principle, features, and methods discussed herein may be applied to any dental application that works with three-dimensional anatomy. It should be noted that the methods and descriptor stacks described herein may also have an application other than in the digital dental industry.

It should also be noted that for illustrative purposes, the various exemplary methods and systems presented herein may be described in connection with a single tooth of a patient. However, such exemplary methods can be implemented with another type of tooth within a patient such as molars, canines, premolars, etc. as well as posts, gaps, and gum structures. For example, an incisor tooth may be illustrated in a single tooth descriptor method, and the same method can also be performed with premolars, molars teeth. In some embodiments, similar ideology may apply to other methods and descriptor stacks. Additionally, the same method can be applied for multiple teeth, dental arch, occlusal or bite pattern, soft tissue structures, and other dentition and dental structures.

The present method and system preferably uses one or more graphical processing unit (GPU) to expedite the CAD design. The present method operates in such a way that 3D files can be converted into 2D matrixes which can easily be passed to one or more GPU for further processing. Since the 3D rendering operator transforms matrixes into 2D images, using GPUs can significantly expedite the dental file segmentation process. In particular, as GPUs are specialized to work with image files, unlike CPUs which are more multi-purpose and operate in parallel rather than sequentially, GPUs are capable of accomplishing tasks much faster. A graphics processing unit (GPU) can thereby be used to accelerate and optimize processing of 2D matrixes and machine learning algorithms for pattern matching. Machine learning techniques, for example those that use neural networks, are also particularly well suited for processing on a GPU because the GPU is typically much more powerful than a typical central processing unit (CPU). It is therefore expected that using GPUs to cluster hundreds of thousands of files will be significantly faster than using CPUs, accomplishing in seconds what would take days for a CPU, and could thus achieve results that are unattainable through current CPU processing.

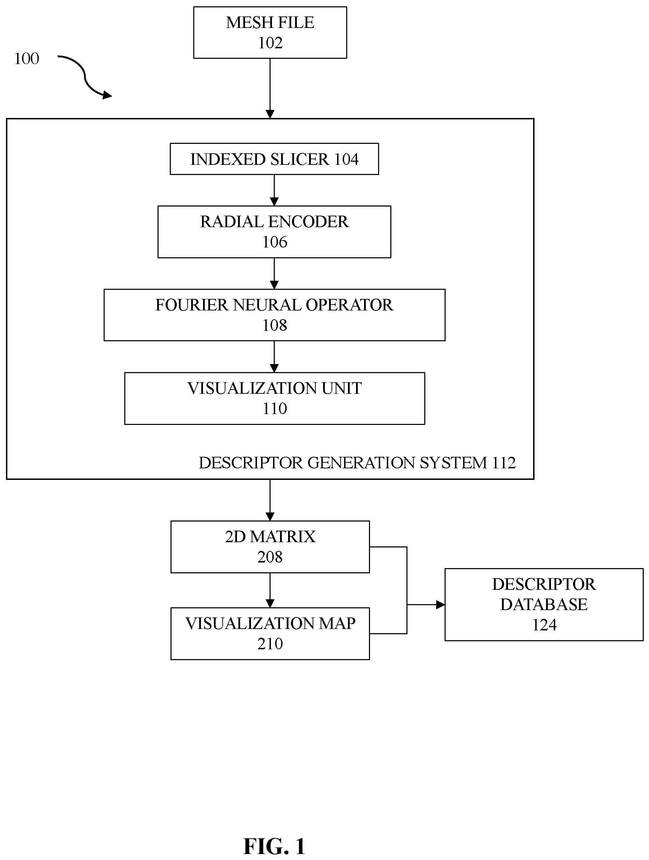

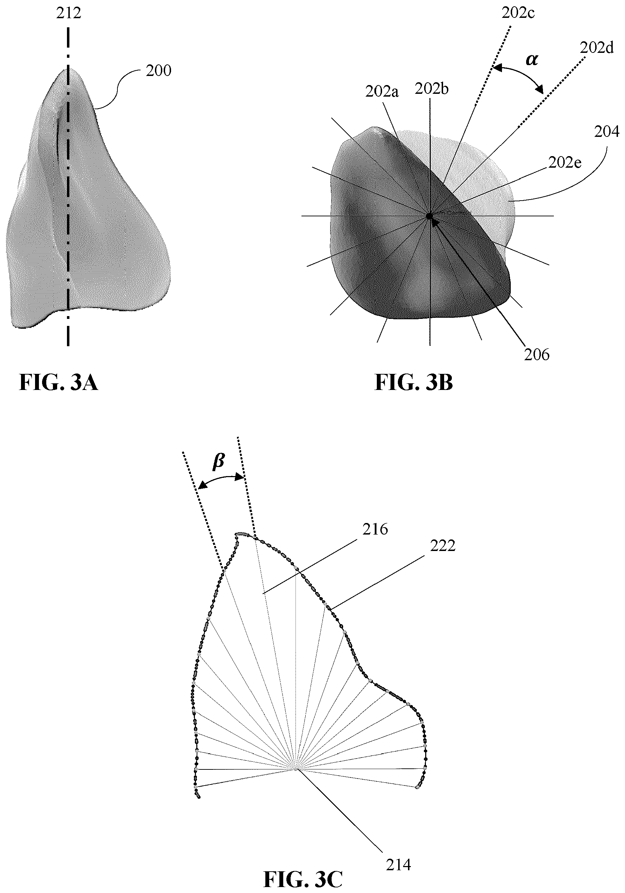

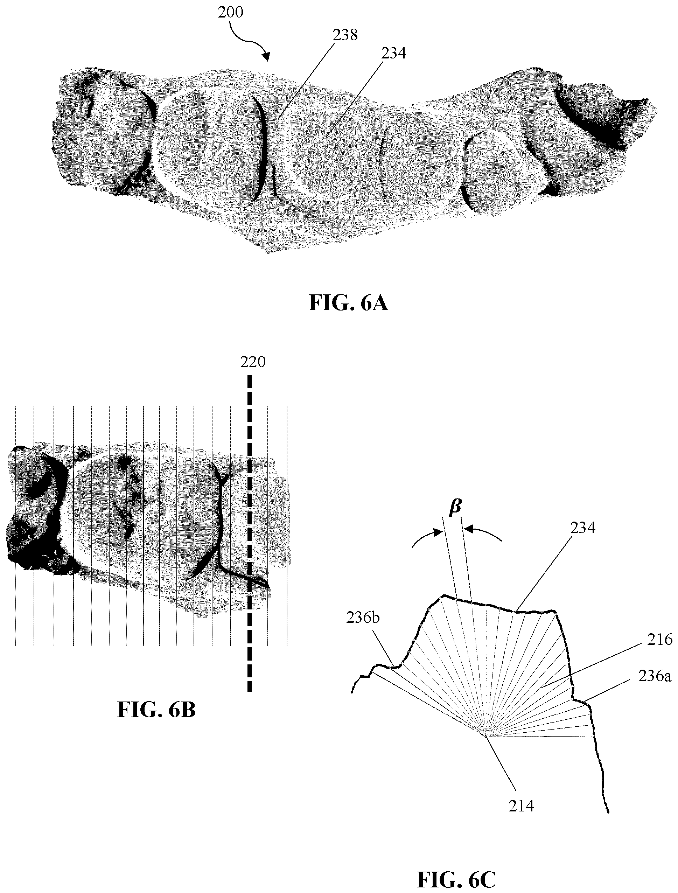

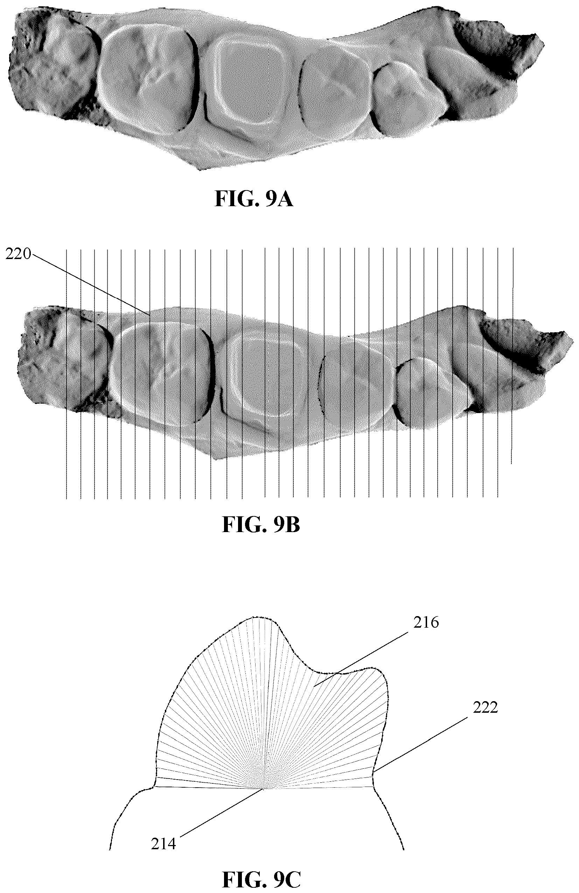

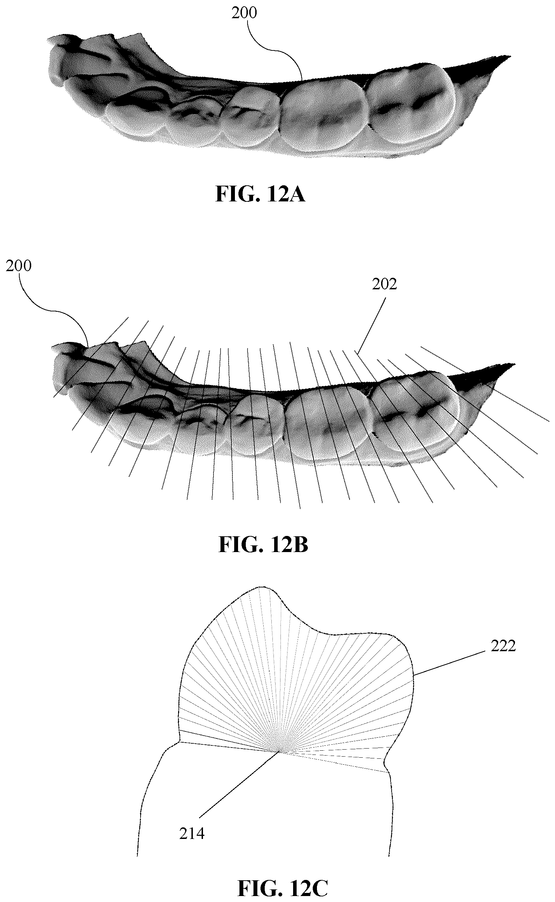

FIG. 1 illustrates an example of a dental file segmentation system 100 in a component diagram. The descriptor generation system 112, in accordance with an embodiment, comprises an indexed slicer 104, a radial encoder 106, a Fourier neural operator 108, and a visualization unit 110 or graphical user interface to image the file format produced by the description generator system 112. The indexed slicer 104 receives an STL or mesh file 102 as an input source and at Fourier neural operator 108 the dental file segmentation system 100 generates the 2D matrix 208 or 2D array as an output. The mesh file 102 of a dental object can be obtained by one of a variety of imaging systems used to collecting imaging data on dental features. A variety of dental cameras are known, and the images received by the cameras can be mosaiced together to create a dental image, which can be either collected as a mesh or STL file, or converted into a mesh file using known software methods. The resulting 2D matrix 208 or array structure may differ based on the methods it is used for, and visualization unit 110 can provide a visualization map 210 as an image output. The image output can be of any standard image file format including but not limited to JPEG, GIF, PNG, TIFF, PSD, PDF, EPS, AI, INDD, RAW, etc. Different descriptors may also be generated using the descriptor generation system 112, including, for example a single tooth descriptor method 1 (see FIGs. 3A-3E), a single tooth descriptor method 2 (see FIGs. 6A-6E), an arch descriptor method 1 (see FIGs. 9A-9E), an arch descriptor method 2 (see FIGs. 12A-12E) and a full arch descriptor. In an embodiment, the present system can use five different mesh file autoencoders to process a full dental arch mesh file digital impression file. The five mesh files can be processed as described, and the resulting five 2D matrix files or arrays can be stacked to describe the full dental features which the corresponding 3D mesh file referred. In this way a highly compact dataset may be derived from the dental descriptors that describe the full dental arch in sufficient detail for modeling, planning, and tracking dental features. In some embodiments, a bite pattern and a bite registration descriptor matrix may be provided for better bite analysis and comparison. A dental object descriptor database 134 can be used to store the plurality of 2D matrices 208 resulting from the descriptor generation system 112.

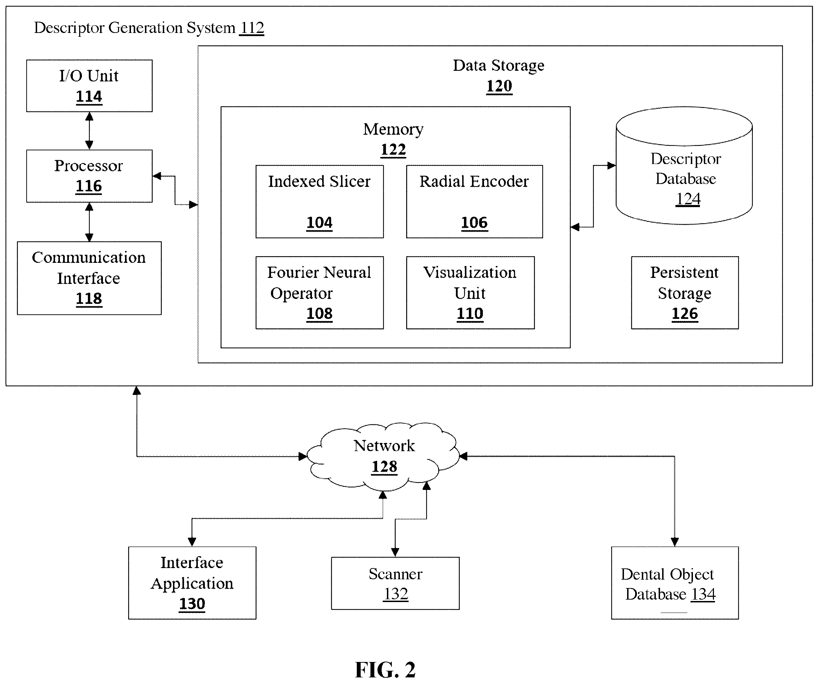

FIG. 2 illustrates, in a schematic diagram, an example of a descriptor generation system 112 in accordance with some embodiments. The descriptor generation system 112 is implemented on a computer or equivalent electronic device connected, either wired or wireless, to an interface application 130, a dental scanner 132 (such as a dental imaging device that produces STL/mesh images), and dental object databases 134 (such as dental records or other data) via network 128. The interface application 130 can be, for example, a dental assessment interface application on a personal computer, a dental assessment device interface, or a mobile device application, generally comprising a graphical user interface, which enables a dental professional to interact with the system. The descriptor generation system 112 can implement aspects of the processes described herein. The descriptor generation system 112 can be implemented on a suitable computer or electronic device and can include an I/O unit 114, a processor 116, using a communication interface 118, and a data storage 120. The descriptor generation system 112 also has a memory 122 storing machine executable instructions to configure the processor 116 to receive files, for example from Input/Output (I/O) unit 114, one or more dental scanner 132 device, or from one or more descriptor databases 124. The dental descriptor database 124 can, for example, be a database comprising a plurality of dental descriptor mesh or mesh files and/or matrix data files that can be called upon for matching, comparison, artificial intelligence or machine learning training or testing sets, or for other comparative purposes.

The descriptor generation system 112 can also include a communication interface 118, and data storage 120. The processor 116 can execute instructions in memory 120 to implement aspects of processes described herein. The descriptor generation system 112 can connect with one or more interface applications 130, dental scanner 132 devices, or dental object databases 134. This connection may be over a network 128 (or multiple networks), either wireless or wired or a combination thereof. The descriptor generation system 112 may receive and transmit data from one or more of these via I/O unit 114. When data is received, I/O unit 114 transmits the data to processor 116. The I/O unit 114 can enable the descriptor generation system 112 to interconnect with one or more input devices, such as a keyboard, mouse, camera, touch screen and a microphone, and/or with one or more output devices such as a display screen and a speaker. The processor 116 can be or comprise, for example, one or more of any one or more type of general-purpose microprocessor or microcontroller, for example, digital signal processing (DSP) processor, integrated circuit, field programmable gate array (FPGA), a reconfigurable processor, or any combination thereof. The data storage 120 can include memory 122, one or more dental descriptor database(s) 124 containing a plurality of dental object 2D matrix representations along with their descriptor class/subclass, and one or more persistent storage 126. Memory 122 may include a suitable combination of any type of computer memory that is located either internally or externally such as, for example, random-access memory (RAM), read-only memory (ROM), compact disc read-only memory (CDROM), electro-optical memory, magnetooptical memory, erasable programmable read-only memory (EPROM), and electrically-erasable programmable read-only memory (EEPROM), Ferroelectric RAM (FRAM) or the like.

The communication interface 118 can enable the descriptor generation system 112 to communicate with other components, to exchange data with other components, to access and connect to network resources, to serve applications, and/or perform other computing applications by connecting to one or more network or multiple networks capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g., Wi-Fi, WiMAX), SS7 signaling network, fixed line, local area network, wide area network, and others, including any combination of these. The descriptor generation system 112 can also be operable to register and authenticate users using, for example, a login, unique identifier, and password, prior to providing access to applications, a local network, network resources, other networks and network security devices. The descriptor generation system 112 can also be enabled to connect to different machines or entities over one or more communication interface 118. The data storage 120 may be configured to store information associated with or created by the descriptor generation system 112. Storage 120 and/or persistent storage 126 may be provided using various types of storage technologies, such as solid state drives, hard disk drives, flash memory, and may be stored in various formats, such as relational databases, non-relational databases, flat files, spreadsheets, extended markup files, etc. The memory 122 may also include the indexed slicer unit 104, the radial encoder unit 106, the Fourier neural operator 108, and the visualization unit 110 as described herein.

### Single Tooth Descriptor Method I

FIGs. 3A to 3E illustrate example outputs of components of the dental file segmentation system 100 employing an example of a single tooth description method, in accordance with some embodiments. Processing of three-dimensional single tooth files can be achieved from their native 3D format as created by a dental scanner and converted to a two-dimensional (2D) format. This can be achieved by slicing the three-dimensional tooth into an equally angled slicing plane and converting the slicing data into a matrix for 2D representation of the 3D dental object, as described. Preferably a graphical processing unit (GPU) is used to expedite the CAD design by processing the converted 2D matrixes in one or more GPU to accelerate and optimize processing for pattern matching.

FIG. 3A illustrates an example of a dental object 200 in a native three-dimensional tooth file (e.g., STL file format), obtained as a scanned image of a tooth from one or more dental scanner. The dental object 200 is shown with z-axis 212 which goes through the highest point, centre, or a high point on the tooth. Each mesh file has a bounding box, and the centroid is usually the centroid of that bounding box, or the bounding box can have the same z-axis as the volumetric centroid of the mesh file. The z-axis usually refers to the longest axis out of the axes, and it is through the z-axis that the dental feature to represent is aligned. Generally the z-axis will be assigned through a single tooth from the occlusal plane toward the jaw.

FIG. 3B is an image of the dental object shown in FIG. 3A viewed from the top through the z-axis. The slicing centroid 206 is preferably on the z-axis, but may also be in another location outside of or not explicitly on the z-axis. As shown in FIG. 3B, the dental object is analyzed by the indexed slicer where, in the present example method, the dental object is sliced radially into a plurality of radial portions 204 through the slicing centroid 206. In the embodiment shown, each radial slice will pass through the tooth centroid or z-axis. As shown in FIG. 3B, all radial slicing planes 202a, 202b, 202c, 202d, 202e that are generated have a consistently increasing angle alpha (*α*) such that the difference in angle or degrees from one radial slicing plane to the next is the same. In some embodiments, increasing the number of radial slicing planes in the method will increase the accuracy of the descriptor generation such that the resulting 2D matrix provides more granularity on the 3D shape of the dental object. Each radial slicing plane 202 will generate a cross-sectional view of the dental object 200 through the plane.

As shown in FIG. 3C, the distance from the indexing centroid 214 to the intersection of each cross-sectional point or tooth cross sectional boundary 222 on the circumference of the dental object slice, or the length of each indexing ray 216, may be measured by the radial encoder. The indexing centroid 214 is the centroid of the cross-sectional plane of a single radial slicing plane generated from radial slicing of the dental object through a radial slicing plane. The radial encoder will generate a plurality of indexing rays 216 originating at the indexing centroid 214. The distance between the indexing centroid 214 and the circumference of the cross-section of the radial slicing plane at the edge of the dental object can be generated from the slicing plane to map the circumference of the dental object in the slicing plane. In some embodiments, all the indexing rays will be equally angled in space at an angle beta (*β*) such that the angle between each indexing ray 216 is constant. It should be noted here that the cross section created by the indexed slicer of the dental object from bottom plane in other word the one can have infinite beta angle. Increasing the number of indexing rays generated will provide more detail about the dental object, for example the tooth anatomy, by capturing more data points around the tooth cross-section circumference. However, more circumferential data points as provided by the distances of the plurality of indexing rays will increase the 2D matrix file size, accordingly this should be kept in mind when determining the number of indexing rays required to provide adequate precision required for the desired purpose.

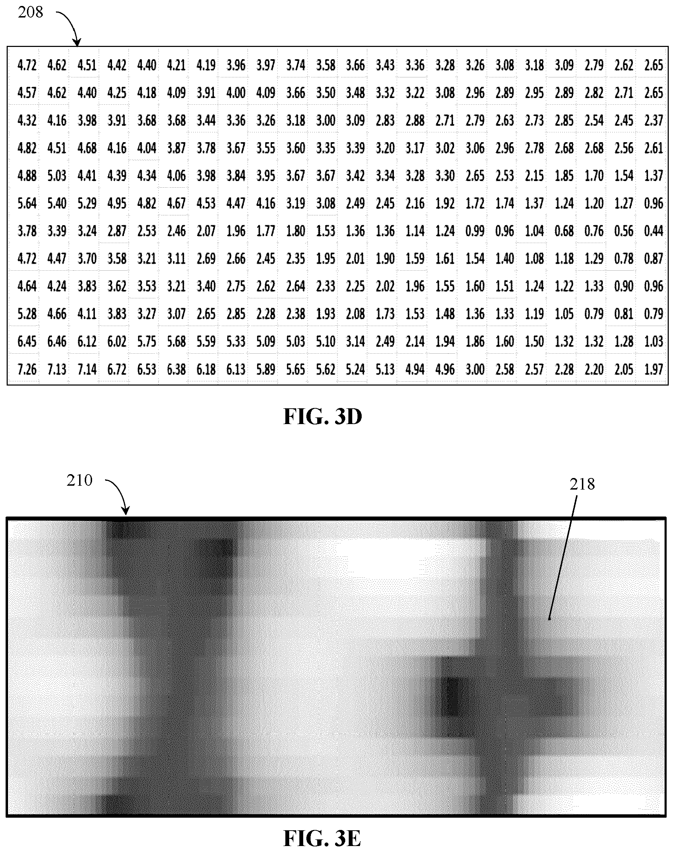

FIG. 3D illustrates an example of a radial encoder output as a 2D matrix 208. Once generated, 2D matrix 208 is then generated from the radial encoder output using a Fourier neural operator for each radial portion of the tooth dental object shown in FIG. 3B. In particular, for each cross sectional slice, the radial encoder measures the distance from the indexing centroid to the cross sectional boundary and generates a 1D array which describes the particular slice. This is repeated for every slice, and combining all of the 1D arrays will create the 2D matrix using a Fourier neural operator. Other mathematical functions can also be applied to the 1D arrays to reduce the numbers present in those array to a manageable range and aim to reduce the data size by reducing the bite size. In an example, if the indexing centroid is placed a significant distance away from the dental object slice, the neural operator can adjust the values in the 1D matrix by normalizing the offset distance of the indexing centroid. In a specific example, array (102, 103, 100) can be represented by array (2,3,1) with a base of 100. The 1D array (2,3,1) consumes less space as compared to (102,103,100) bytewise, but normalization of the values has not decreases the accuracy of the matrix. One open source method that can be used for such value normalization is Apache parquet. It should be understood that a full matrix describing the whole dental object may have many rows and columns, potentially on the order of hundreds or thousands of rows and columns to provide sufficient granularity for the entire anatomy of the dental object. The data in the 3D matrix represent the actual distance in mm, and the smallest number form all the numbers in the 3D matrix is removed through normalization, the 3D matrix represents the relative difference or dental variation present in the mesh file.

FIG. 3E is an illustration of a visualization map 210 of the radial encoder output as a pixel array. The visualization map 210 of the radial encoder output can be visualized on a graphical user interface as a pixel array. As shown, each entry in the 2D matrix may be converted to a pixel value in a visualization or mapping to create an image or pixel array. In some embodiments, the number of rows is equal to the number of slicing planes generated from the indexed slicer and the number of columns is equal to the number of ray lengths generated in each slicer in the radial encoder. Each element in the matrix represents a distance from the indexing centroid to a cross-section boundary point of that slicing or circumference of the dental object. The output of the Fourier neural operator is a 2D matrix which can further be used in stacking applications as described below. However, the 2D matrix may also be visualized as the pixel array shown an aid to a dentist or lab technician for further analysis. For example, the value of the Neural operator matrix may be mapped to a color mapping method (e.g., rainbow color, black and white shading, etc.). The minimum and maximum values of the matrix may be identified and assigned to a selected color map value extreme point. Thus, the matrix may be represented as a color map. FIG. 3E illustrates an example of a greyscale visualization map 210 of the 2D matrix shown in FIG. 3D. The values in the matrix are represented as shading shown in pixel region 218. Again, a full matrix would correspond to the entire visualization mapping/image such that each conversion of a matrix entry to a colour/shading may be represented as a pixel in the visualization map 210.

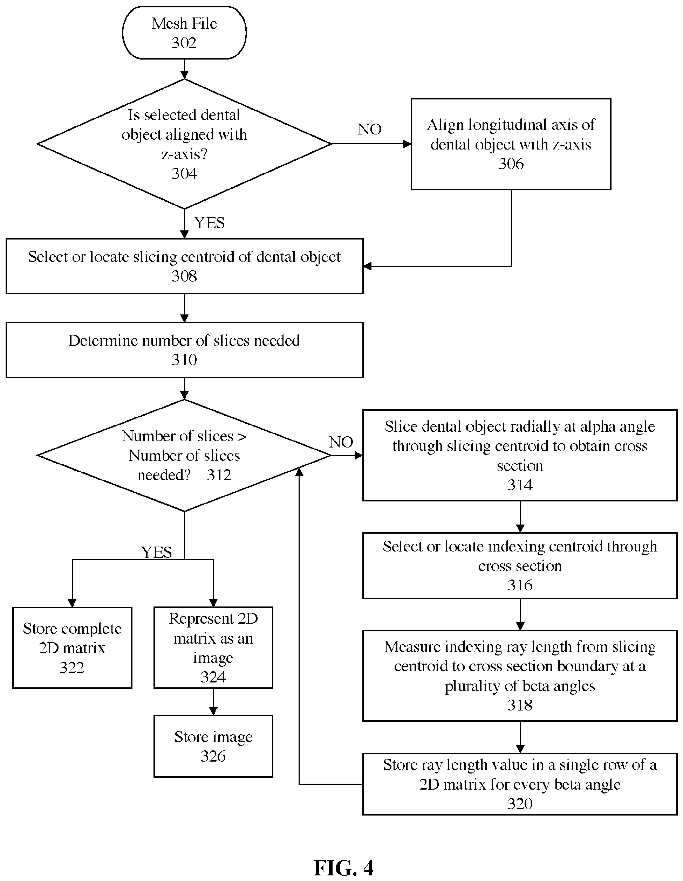

FIG. 4 illustrates, in a flow chart, an example computational of a method of generating a single tooth or dental object descriptor 300, in accordance with some embodiments. The method of generating a single tooth descriptor 300 takes a 3D single tooth mesh file 302 (e.g., in an STL format) as an input. In some embodiments, the file may represent a dental object or dental anatomy such as a tooth as shown in FIG. 3A. In other embodiments, the file may represent a group of teeth that include the tooth for which the descriptor is generated, multiple teeth, dental arch, occlusal or bite pattern, soft tissue structures, or other dentition or dental structure. In some embodiments, the z-axis should be aligned with the anatomy's (e.g., object or tooth) longitudinal axis. The system queries whether the selected dental object is aligned with the z-axis 304. If the selected tooth is not aligned with the z-axis then the anatomy's longitudinal axis is aligned with the z-axis 306. In some embodiments, the system automatically aligns the dental anatomy's longitudinal axis to the z-axis. For example, the system may generate a transformation matrix which moves the center of a bounding box of the input STL to the origin. This aligns the dental anatomy's longitudinal axis aligned with the z-axis. The alignment with the z-axis may be performed to ensure the data is generated and stored uniformly. Once the anatomy or selected tooth is aligned with the z-axis, a slicing centroid, or the centroid of a group of teeth that includes the selected tooth, is located 308. Next, the number of slices needed to ensure that all features are covered is determined 310. For example, if the number of slices is too few, then the space between slices may be large enough so that the cross-sections provided by the plurality of slices does not show a particular anomaly or feature of the anatomy. The step of determining the number of slices through the slicing centroid 310 will determine the quality or granularity of the 2D matrix representation and resulting visualization map of the dental object or tooth. Next, if the number of slices is not greater than the number of sliced needed 312, then the tooth or dental object may be sliced radially at an alpha angle as a plane normal in a clockwise or anti-clockwise direction with the slicing centroid as a plane origin 314. At this point, the process provides a cross-section view for each slice. To do this, the system locates or selects the indexing centroid 316 for each cross-section, and then measures the length of the indexing ray from that indexing centroid to the cross-section boundary 318 in a clockwise or anti-clockwise direction, preferably at an approximately equal beta angle. The measurements comprise indexing ray length values for each slice or slicing plane and can be stored as a single row unit 320. Steps 312 to 320 may be repeated in a loop to obtain single row units for each slice, which can then be stored in a 2D matrix 322 format one after another. The generated 2D matrix may also be stored in a data repository such as a matrix database.

In some embodiments, the method for generating a single tooth or dental object descriptor may be performed for another tooth anatomy for example another incisor, molar, premolar three-dimensional teeth. The matrix obtained for each output of method may be stored in as a 2D matrix 322, optionally in a matrix database. This method also allows the storage of the plurality of created 2D matrices in an image format 324 or as an image 326, such as a visualization map. The image format or visualization map may be stored by, for example, representing the maximum and the minimum value in the matrix to a chosen color extreme values, representing an image file for that corresponding tooth matrix 324. It should be noted that the image visualization map file format is not limited to any single file format, which can include but is not limited to JPEG, GIF, PNG, TIFF, PSD, PDF, EPS, AI, INDD, RAW, etc. The 2D matrix, which can be represented as one or more image or visualization map images, can be stored 326 with the same method described above for the tooth database and can be linked with each other.

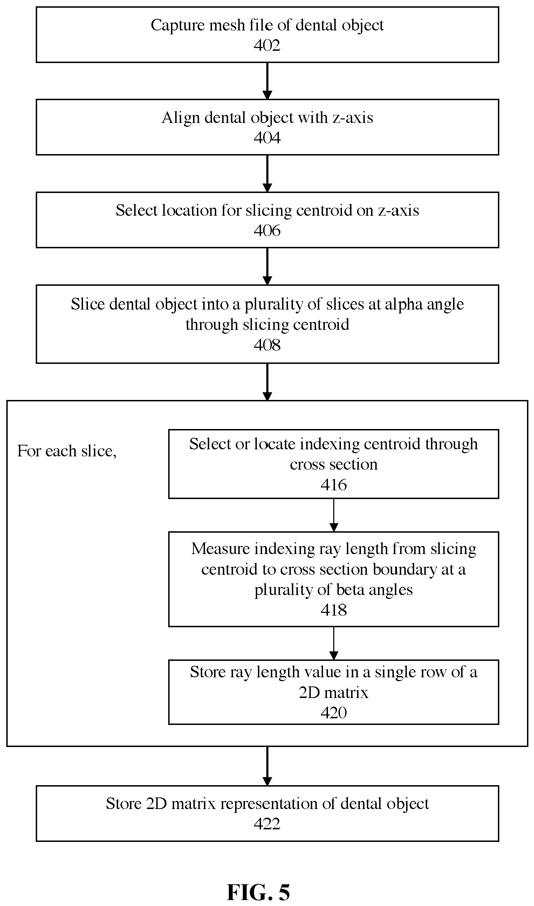

FIG. 5 is an example method of dental file segmentation and creation of a 2D representation for a dental object. First the mesh file of a dental object 402 is captured. Then the dental object is aligned with the z-axis 404 The location for the slicing centroid on the z-axis is then selected 406, and the dental object is sliced into a plurality of slices at alpha angle through the slicing centroid 408. For each slice, an indexing centroid is selected or locate through cross section 416, a plurality of indexing ray length are measured from the slicing centroid to cross section boundary at a plurality of beta angles 418, and the identity or location and length of each ray length value is stored in a single row of a 2D matrix 420. The stored or complete 2D matrix representation is then a descriptor including a value mapping of the dental object 422.

### Single tooth Descriptor Method II

FIGs. 6A to 6E illustrate an example method of generating a dental object descriptor employing an example of another single tooth description method in accordance with some embodiments. The method of generating a single tooth descriptor uses components of the descriptor generation system and employs another example of a single tooth or dental object description method. In particular, this method demonstrates an embodiment of related processing of three-dimensional single tooth files from their native 3D format to a 2D format. This method is different than the previously discussed method based on the input. A dental professional may scan a group of teeth or dentition, and not just a single tooth, as an input to the method. A group of teeth may be called a "bitewing" if it is three or four teeth together, a quadrant if is almost or half of a dental arch, or a dentition if referring to any other grouping of dental anatomy or structure. The entirety of the upper or lower set of teeth may be called the "dental arch".

FIG. 6A illustrates an example of a dental object 200, which in this case is a quadrant, in a three-dimensional tooth anatomy image. The image can be received by a scanner as a set of photogrammetry or camera images and converted into an STL file format, also referred to as a mesh file format, or may be scanned directly in an STL file format by a dental scanner. In some embodiments, a tooth or teeth for which a descriptor is to be generated may be located in this anatomy. The method shown can be utilized for the design of a crown, or fabricated crown, that can fit on post 234. Post 234 is the remainder of a tooth that has been shaved down during dentistry, and the present method is used to visualize the post and surrounding gum tissue as well as neighbouring teeth in order to design a suitable crown that will fit onto the post 234. As shown, gumline margin 238 around the post will be the seating surface for the final crown, and it is important that the crown edge, or crown margin, be designed to fit the gumline margin.

FIG. 6B illustrates an example of a portion of the three-dimensional quadrant that is cut in longitudinal slicing planes in such a manner that it shows one tooth and 25% of both of its adjacent teeth (without slicing planes). A single longitudinal slicing plane 220 is also shown. One way of slicing the 3D STL or mesh file is to image that a slicing centroid is placed at an infinite distance away from the dental object, such that the slicing planes are substantially parallel. This is in contrast to the radial slicing planes when the slicing centroid is inside or near the 3D image of the dental object. Different indexed slicers may be used. As shown in FIG. 6B, all slices that will be generated may be approximately equally spaced. The more slices there, then the better the accuracy that can be achieved. Each slicing plane may generate a cross-section view.

FIG. 6C illustrates an example of a radial encoding of a cross section of a post 234 through a slicing plane, as described above. The indexing centroid location can be selected depending on what features of the dental object are desired for imaging or focusing on or on the selected array value mapping algorithm. The user can also have the freedom to select the index centroid, or the indexing centroid can be automatically selected by the system either by dental object shape, descriptor, or by the descriptor dental file segmentation algorithm. The distance from an indexing centroid 214 to an endpoint of an indexing ray 216 on a tooth perimeter in the slicing plane may be measured using a radial encoder. The radial encoder may generate the indexing rays of segments in the slicing plane which maps the distance from the indexing centroid 214 to the circumference of the dental object cross-section generated from the slicing plane to the indexing centroid 214. In some embodiments, all the indexing ray 216 segments may be approximately equally angled in space, meaning that they are separated at the same angle, shown here as angle *β*. It should be noted here that the cross section created by the indexed slicer can also be measured from the bottom plane or below, for example, by placing the indexing centroid 214 at a large or infinite distance from the dental object circumference to provide essentially an infinite beta angle such that the indexing rays 216 are substantially parallel. The more ray segments generated, then the better details will be provided about the tooth anatomy. However, file size should also be kept in mind with respect to how much ray segments are feasible. The sides of the margin or margin shoulders 236a, 236b are shown which provide geographical structure of the post and surrounding gum tissue such that the crown can be designed to securely but comfortably fit both the post 234 and the gum.

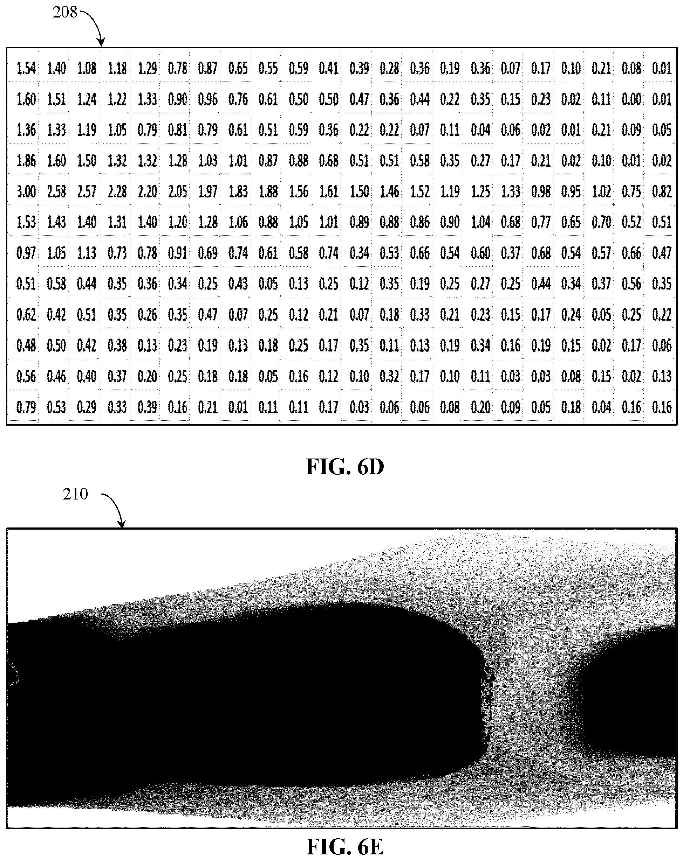

FIG. 6D illustrates another example of the output 2D matrix 208 of the Fourier neural operator for the plane of the tooth, gum features, and post shown in FIG. 6B as a data set from each slicing plane. A Fourier neural operator may generated a 2D matrix 208 from the radial encoding. Only the matrix values for each slicing plane is shown in 2D matrix 208 for ease of presentation. It should be understood that a full matrix may have many rows and columns, on the order of hundreds or thousands of rows and columns, to provide sufficient granularity for the entire anatomy. Each entry in the matrix may also be represented as a pixel value in a visualization mapping/image. In this example, the number of rows of 2D matrix 208 is equal to the number of slicing plane generated from the indexed slicer, and the number of columns is equal to the number of indexing rays generated in each slice by the radial encoder. Each element of the 2D matrix 208 represents the distance from the indexing centroid to the cross-section boundary of the dental object in that slicing plane. The output of the Fourier neural operator is the desired 2D matrix 208 which can further be used in stacking applications.

FIG. 6E illustrates an example of a visualization mapping/image using a black and white greyscale mapping. The 2D matrix may be visualized as a visualization map 210, which can be useful to dentists or lab technicians for further analysis. In some embodiments, such visualization may be generated via a mapping of the values of the neural operator matrix to a rainbow color, black and white shading or greyscale, or any other color mapping methods. The minimum and maximum values of the 2D matrix may be identified and assigned to the selected color map value extreme points. The values in the 2D matrix are represented as shading shown in each slicing plane. Again, a full matrix would correspond to the entire visualization mapping/image such that each conversion of a matrix entry to a colour/shading may be represented as a pixel in the visualization map 210.

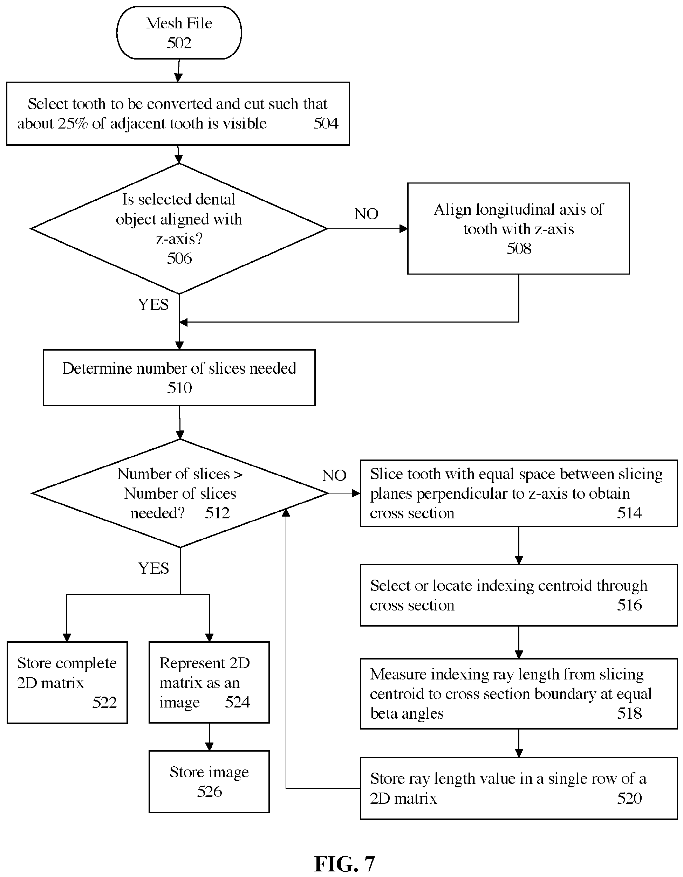

FIG. 7 illustrates, in a flow chart, another example of a method of generating a single tooth or dental object descriptor, in accordance with some embodiments. The method begins with taking a three-dimensional quadrant STL file 502 as an input, either as a raw STL or mesh file from a dental scanner or as an STL or mesh file created from a different type of scan, such as a visual scan or mosaic of images, video, or photographs. For example, the 3D quadrant may be similar to that shown in FIG. 6A. Next, the targeted tooth to be converted is selected in 504. In some preferable embodiments, the selected toot is converted and cut from the quadrant in a manner where 25% of the adjacent teeth are visible. The system then queries whether the longitudinal axis of the selected tooth is aligned with a z-axis 506. If the z-axis is not aligned, then the anatomy (e.g., tooth) is aligned with the z-axis 508. In some embodiments, the system automatically aligns the dental anatomy's longitudinal axis to the z-axis. For example, the system may generate a transformation matrix which moves the center of a bounding box of the input mesh file to the origin. This aligns the dental anatomy's longitudinal axis aligned with the z-axis.

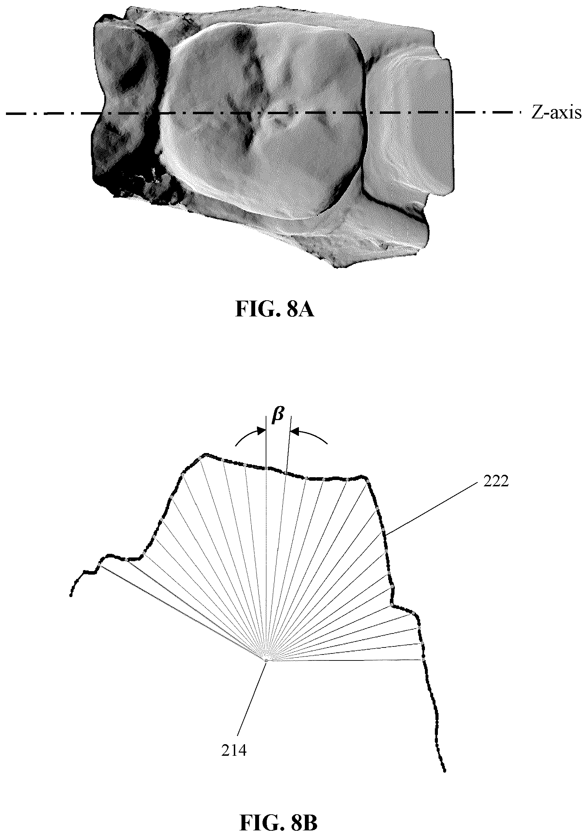

The number of slices needed to ensure all features are covered is then determined 510. For example, if the number of slices is too few, then the space between slices may be large enough so that their cross-sections do not show an anomaly or feature of the anatomy. This step will determine the quality of the 2D matrix and the resulting image or visualization map. The method may now go into a loop based on condition 512 such that the number of slices obtained is equal or greater to the number of slices needed. If the number of slices is not greater than the number of slices needed 512 then the tooth is sliced at approximately equal spacing, as a plane normal to the z-axis 514, for example in left to right direction, to obtain a cross-section view for each slice. It should be noted that the slicing can be from right to left as long as all teeth are sliced consistently in the same manner (i.e., always from left to right, or from right to left). Next, a slicing centroid is selected or located 516 for each cross-section, and the radial lengths between the slicing centroid and a cross-section boundary point are measured (see FIG. 8B) 518. It should be noted that the same features can be obtained in a clockwise or with an anti-clockwise direction; the only difference is it will be in opposite direction as compared to one obtained from clockwise direction at an approximately equal beta angle. It is noted that performing the same algorithm with the same alpha and beta angles, including the same number of slices and rays creates a normalized value map for the 2D matrix to enable descriptor comparison across many different dental objects. The output ray length values for a slice is provided, and the values can be stored as a single row unit 520 in a 2D matrix. By running this loop (steps 512 to 520) single row units for each slice can be obtained and stored in the matrix format one after another. The complete 2D matrix comprising all of the ray lengths and locational attributes on the dental object may be stored in the matrix database 522. The method may be performed for another tooth anatomy for example another targeted incisor, molar, or premolar three-dimensional teeth. The matrix obtain can for each output of method 600 may be stored in the matrix database. The 2D matrix may also be stored in an image format 524 as a visualization map, which may be generated by representing the maximum and the minimum values in the 2D matrix to chosen color or hue extreme values and saving an image file 526 corresponding to the tooth matrix. The image file format is not limited to any single file format and can be stored in an image repository 526. One or more of the tooth or dental object database and the descriptor database may also be linked with the image repository.

FIGs. 8A and 8B illustrate stages of obtaining slices of a tooth, in accordance with some embodiments. FIG. 8A illustrates alignment of a dental object with a single tooth and adjacent teeth with an example a z-axis position for the dental anatomy or dental object. Aligning the dental anatomy's axis to the z-axis helps ensure that the data is collected and stored uniformly. FIG. 8B illustrates the indexing of one cross section plane of a tooth, with a plurality of indexing measurements taken from the indexing centroid to the tooth cross sectional boundary 222 at a plurality of beta (β) angles.

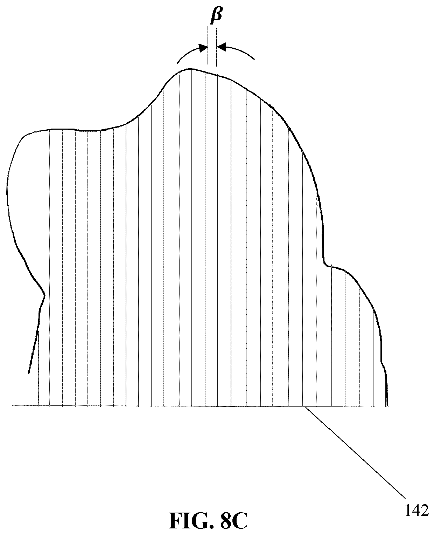

FIG. 8C is an image of a tooth which is processed by an indexing centroid positioned at a relatively infinite distance from the tooth inside a bounding box. By positioning the indexing centroid far from the tooth the indexing rays defined by the ray from the indexing centroid to the cross sectional boundary will be substantially parallel, and the array value mapping can be done from the bottom of the bounding box 142. In addition, the length of the indexing rays will be longer than if the centroid is closer to the tooth or dental object, however the distances can be normalized for processing using a Fourier neural operator or other normalization procedure. As such, instead of slicing radially, the teeth may be sliced in a parallel manner as shown, and the ray length distances can be recorded in the 2D matrix as starting from the bottom of the bounding box 142. Other ways that can be used change the centroid position target the different dental anatomy features like margin, occlusal plane, etc. Preferably all substantially parallel slices that are generated will be approximately equally spaced to provide consistency and continuity across the resulting 2D matrix representations of the tooth or dental object. The more slices are done then the better the accuracy of the 2D matrix that can be achieved, however the more data is collected, as each slicing plane generates its own unique cross-section view and results in an individual row in the matrix. As such, the algorithm is tailored to provide a balance between data file size and surface feature granularity and accuracy. If the dental object or anatomy is sliced in parallel, there is no need to locate a teeth centroid. However, consistency in slicing parallel with regard to distance between slicing planes, is preferred so that similar dental anatomy can be compared, where the dental file segmentation algorithm used is consistent across dental objects having the same descriptor classification.

### Arch Descriptor Method I:

FIGs. 9A to 9E illustrate examples outputs of components of the descriptor generation system employing an example of an arch description method, in accordance with some embodiments. This method is related processing of a three-dimensional dental arch file from its native 3D format to a 2D format. This method can also function with different dental anatomies such as a bitewing or a quadrant as shown. The term 'bitewing' can be used if the dental object is three to five teeth together, and the term 'quadrant' can be used if the dental object is half or almost half of the dental arch. The same notation applies to both upper and lower anatomy. FIG. 9A illustrates a dental quadrant as the dental object in a mesh or STL file format. As shown in FIG. 9B, all slices that will be generated are approximately equally spaced, and an increase in the slices improves accuracy. A longitudinal slicing plane 230 is also shown, which is a single slicing plane through the dental object which is parallel or substantially parallel to the other slicing planes through the dental object. This method is similar to the previously discussed method of a single tooth descriptor method as the methods both slice the dental anatomy in parallel. Each longitudinal slicing plane 230 will generate a cross-sectional view of the dental object.

FIG. 9C shows a single parallel slicing plane and dental object cross sectional boundary 222 through that slicing plane. The distance from the indexing centroid 214 to endpoints of the perimeter of the cross-section view are measured by a radial encoder. The radial encoder will generate a plurality of indexing rays 216 at a plurality of angles from a first indexing ray, where the angular distance between indexing rays is the beta (β) angle. A smaller beta angle will provide increased resolution of the indexing measurement, however will generate more data. A balance between greater resolution of the cross-sectional boundary and the amount of data desired can be selected by changing the size of the beta angle during radial encoding. The radial encoder then determines the length of each of cross-sectional ray generated from the slicing plane to the indexing centroid 214 and records the locational information of the indexing ray 216 (slicing plane, β-angle, etc.) along with the length of the indexing ray in a matrix. The greater the number of ray segments that are generated, the better the details about the tooth anatomy will be. However, file size requirements may result is limiting the number of ray segments. In some embodiments, all indexing ray segments will be separated by the same β-angle, however it is understood that the β-angle can be changed during indexing, for example by increasing the β-angle near the occlusal surface where more measurements provide improves occlusal surface resolution which can be beneficial for dental prosthetic design. Although the indexing centroid 214 is shown here inside the cross-sectional plane of the dental object, it is noted that the indexing centroid 214 can also be positioned outside the dental object plane, or even at a near infinite distance from the dental object to provide a substantially parallel set of indexing ray, and the cross sectional length created by the indexed slicer from a bottom plane can be used to measure ray length at an infinitely small beta angle. If the cross sectional plane of the dental object is sliced substantially in parallel in this way, the indexing centroid effectively becomes an indexing plane, and the length of the indexing rays can be determined from the indexing plane to the cross-sectional boundary 222. The matrix may then be generated from the radial encoding by the Fourier neural operator.

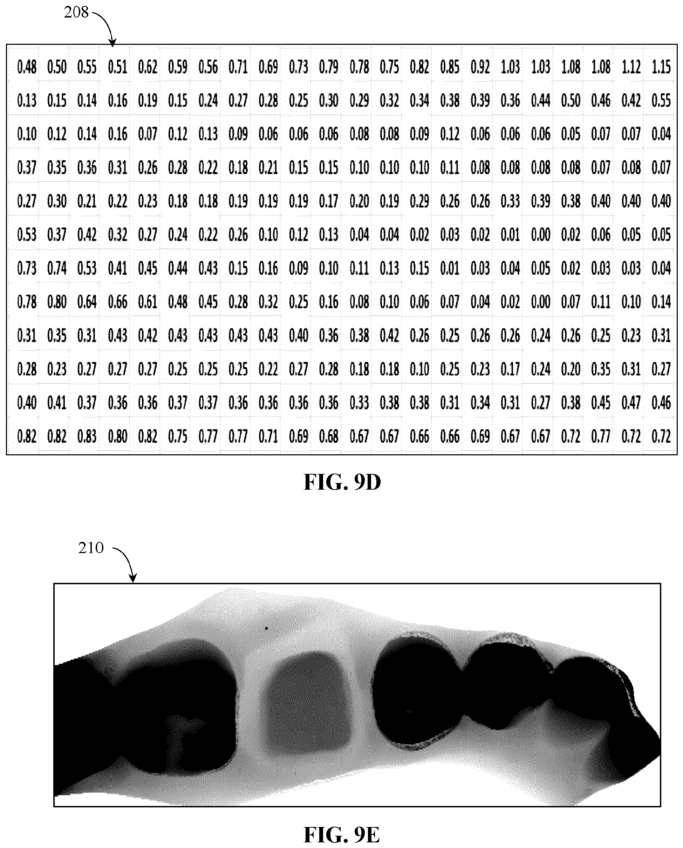

FIG. 9D shows an example of an output 2D matrix 208 of the Fourier neural operator for the dental object tooth shown in FIG. 9A. Only the matrix values for the cross-sectional plane of the dental object are shown in 2D matrix 208 for ease of presentation. It should be understood that a full matrix describing a single dental object may have many rows and columns, perhaps in the order of hundreds or thousands, to provide sufficient granularity for the entire dental object anatomy. Each entry in the matrix may be used as a pixel value in a visualization mapping/image. In some embodiments, the number of rows is equal to the number of slicing planes generated by the indexed slicer, and the number of columns is equal to the number of ray lengths generated in each slice by the radial encoder. Each element of the 2D matrix 208 shown represents the distance from the indexing centroid to the cross-section boundary of the indexing ray, as shown in FIG. 9C. The output of a Fourier neural operator is the 2D matrix 208 which can further be used in stacking applications described below.

FIG. 9E illustrates an example of a matrix visualization map 210 using black and white scale (greyscale) mapping. The 2D matrix can be visualized to assist dentists and/or lab technicians for further analysis. The values of the Neural operator matrix may be mapped to, for example, rainbow colour, black and white shading, or any other colour mapping method. The minimum and maximum values of the 2D matrix may be identified and assigned to the selected colour map value extreme points to generate the colour map. The values in the 2D matrix can also be represented as greyscale shading shown in visualization map 210. A full matrix would correspond to the entire visualization mapping/image such that each conversion of a matrix entry to a colour and/or shading may be represented as a pixel in the visualization map 210.

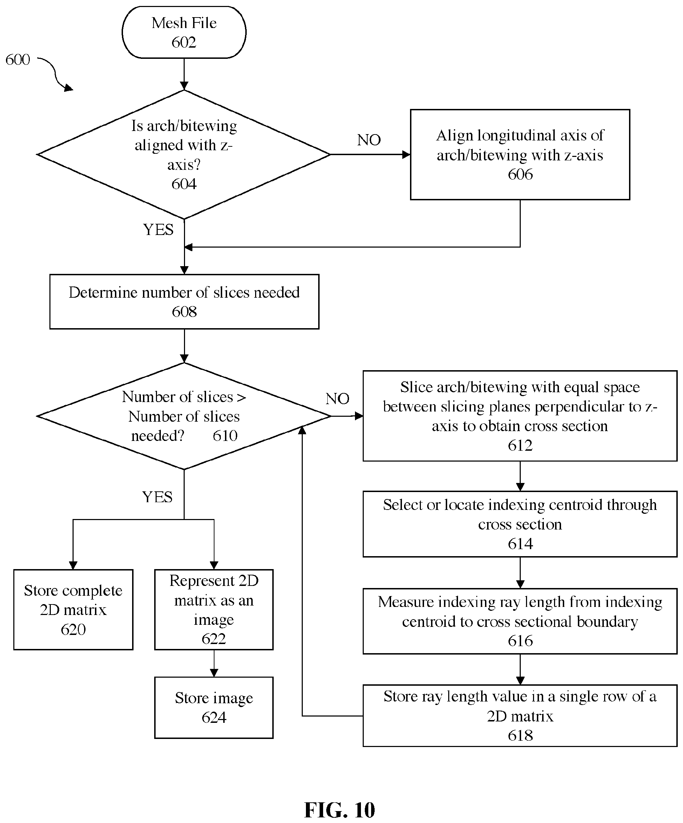

FIG. 10 illustrates, in a flowchart, an example of a method of generating a dental arch descriptor method, in accordance with some embodiments. The dental arch descriptor method begins by taking a three-dimensional mesh file 602 of a dental object as an input. The mesh file 602 representing a quadrant, arch, or bitewing, is a triangulated representative of the image of the quadrant file. The system queries whether the arch or bitewing is aligned with the z-axis of the image 604, and if not, the z-axis of the mesh file is aligned with the anatomy's longitudinal axis 606. In some embodiments, the system automatically aligns the dental anatomy's longitudinal axis to the z-axis. For example, the system may generate a transformation matrix which moves the center of a bounding box of the input STL or mesh file to the origin. This aligns the dental anatomy's longitudinal axis with the z-axis 606. The number of slices needed to make sure all features are covered is then determined 608. For example, if the number of slices is too few, then the space between slices may be large enough so that their cross-sections do not show an anomaly or feature of the anatomy. This step of determining the number of required slicing planes 608 determines the quality of the 2D matrix datafile produced by the method, and its accuracy relative to the original image file of the dental object. A recursive loop is then entered based recursive slicing the dental object, here an arch or bitewing, along a plurality of slicing planes, each slicing plane having an incremental distance to the next slicing plane, until the number of slicing planes or slices reaches the desired number. If the number of slices is not greater than the number of slices needed 610 then the arch or bitewing is sliced at approximately equal spacing, as a plane normal to the z-axis 612 to obtain a cross-section view for each slice. In this example, the arch or bitewing is sliced with equal space between slicing planes perpendicular to z-axis to obtain a cross section 612. The slicing operation can go from left to right, right to left, or in any other reasonable direction through the dental object. It should be noted that the slicing can be from right to left as long as all teeth are sliced consistently in the same manner (i.e., always from left to right, or from right to left). One reason for standardizing the slicing algorithm for a particular class of dental descriptor is to provide a basis for comparison of the resulting 2D matrix to other 2D matrices with the same descriptor. Next, for each slicing plane, an indexing centroid is selected or located for each cross-section 614, and for each cross-section or slice, a plurality of indexing rays are assigned from the indexing centroid to the cross-sectional boundary of the slicing plane, and each indexed ray length is measured from the indexing centroid to the cross sectional boundary 616. The radial or indexing ray lengths between the indexing centroid and a cross-sectional boundary point on the slicing plane are measured in a clockwise or anti-clockwise direction, with an increasing beta angle between each indexing ray. It should be noted that the same features can be obtained with the anti-clockwise direction as a clockwise direction; the only difference is it will be in opposite direction as compared to one obtained from clockwise direction, at an approximately equal beta angle. As previously mentioned, standardization of the algorithm for converting the dental object into a 2D matrix file enables easier comparison and matching between 2D matrix files in the same descriptor class. The output measured ray length values for each slicing plane can then be stored as a single row unit 618 in a 2D matrix. By running this loop (steps 610 to 618) single row units for each slice can be obtained and stored in the 2D matrix datafile format one after another. This resulting 2D matrix may be stored 620 for example in a matrix database with other 2D matrices of dental objects. The same or similar method of generating a 2D matrix descriptor of a dental object may be performed for any another tooth anatomy or dental object, for example a single tooth such as a targeted incisor, molar, or premolar three-dimensional teeth. The 2D matrix may be visualized or converted into an image 622, for example by setting color or hue values for each range of numerical indexing ray length values, and the same can be stored in an image file format 624. A visualization or image representation of the 2D matrix may be generated, for example, by representing the maximum and the minimum values in the 2D matrix to chosen color or shading extreme values and saving the image file corresponding to the 2D matrix of the dental object. The image file format is not limited to any single file format and can be stored in an image repository, which can be in the same or different location as where the related 2D matrix is stored. The dental object database may also be linked with or connected to the image repository.

### Arch Descriptor Method II:

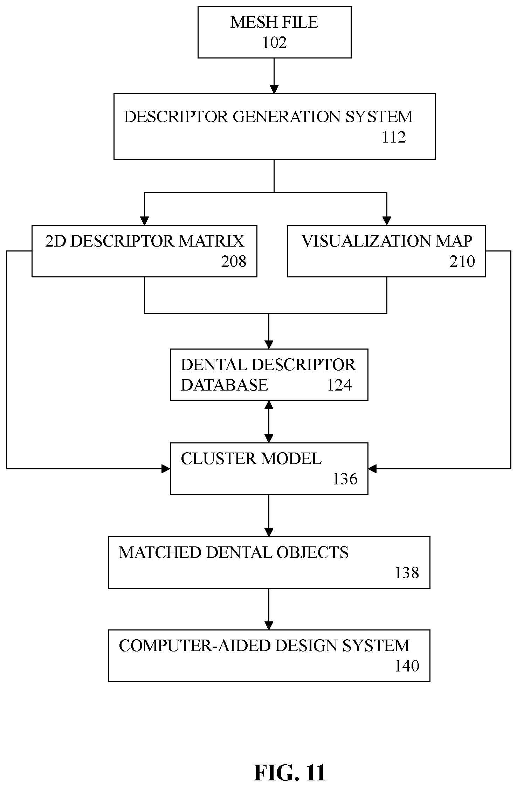

FIG. 11 is an example system flow diagram for designing a dental prosthetic. A mesh file 102 of a dental object is processed by descriptor generation system 112, which performs a dental file segmentation on the dental object to provide a 2D descriptor matrix 208 of the dental object. In the case where a crown is desired, the dental object can be a single tooth or post, or a single tooth or post with all or parts of adjacent teeth, including the gumline around the tooth. The dental file segmentation can also produce a visualization map 210 of the 2D matrix, which can be displayed on a graphical user interface. The 2D descriptor matrix 208 and/or the visualization map 210 of the dental object is then compared to other 2D descriptor matrixes and/or visualization maps of dental objects in a dental descriptor database 124. The dental descriptor database 124is a data repository of dental objects classified using 2D matrix descriptors and classification to identify which tooth or teeth are described by the dental object and 2D descriptor matrix 208 and/or the visualization map 210 thereof. A cluster model 136 is then used to match the 2D descriptor matrix 208 and/or visualization map 210 of the dental object to one or more similar dental objects with a similar 2D descriptor matrix 208 and/or visualization map 210 in the dental descriptor database 124. Once one or more matches is found 138, a computer-aided design system 140 can be used to design a dental prosthetic based for the dental object based on a dental prosthetic previously designed for the matched dental object.

FIGs. 12A to 12E illustrate examples outputs of components of the descriptor generation system employing another example of an arch description method, in accordance with some embodiments. This method is related processing of a three-dimensional dental arch file from its native 3D format to a 2D format. This method can also function with different dental anatomies such as a bitewing (if it is three or four teeth together) or a quadrant (it is almost half of the dental arch, and this notation applies to both upper and lower anatomy. FIG. 12A illustrates an example of a quadrant dental object 200 in a mesh or STL file format. FIG. 12B illustrates a quadrant or dental object 200 which has been passed through an indexed slicer. In this case, it is sliced radially to produce a plurality of slicing planes 202. In this embodiment each slicing plane 202 passes through an arch centroid, which in this case is outside and at a distance from the dental object. Each slicing plane 202 generated by the indexed slicer is at an approximately equal angle to the next slicing plane. Increasing the number of slices increases the accuracy.

FIG. 12C shows a single slicing plane and dental object cross sectional boundary. Each slicing plane will generate a cross-sectional view, and the distance from an indexing centroid 214 to endpoints of the perimeter of the cross-section view, or the cross sectional boundary 222, are measured by a radial encoder. The indexing centroid 214 can be considered to be the centroid of the cross-section generated from the slicing plane, however the indexing centroid can be anywhere inside or outside the dental object, providing that indexing of each indexing ray is done relative to the selected indexing centroid and cross sectional boundary 222. The radial encoder will generate the ray segments which maps the distance from cross sectional boundary 222 in the slicing plane to the indexing centroid 214. In some embodiments, all ray segments will be approximately equally angled in space, meaning that the angle between each indexing ray is constant, however this is not necessary as previously described. In particular, the cross section created by the indexed slicer can also be measured from a bottom plane or indexing plane at a distance away from the slicing plane to provide a substantially infinite beta angle. The greater the number of indexing ray segments that are generated, the better the details about the tooth or dental object anatomy will be. However, file size requirements may result is limiting the number of indexing ray segments.

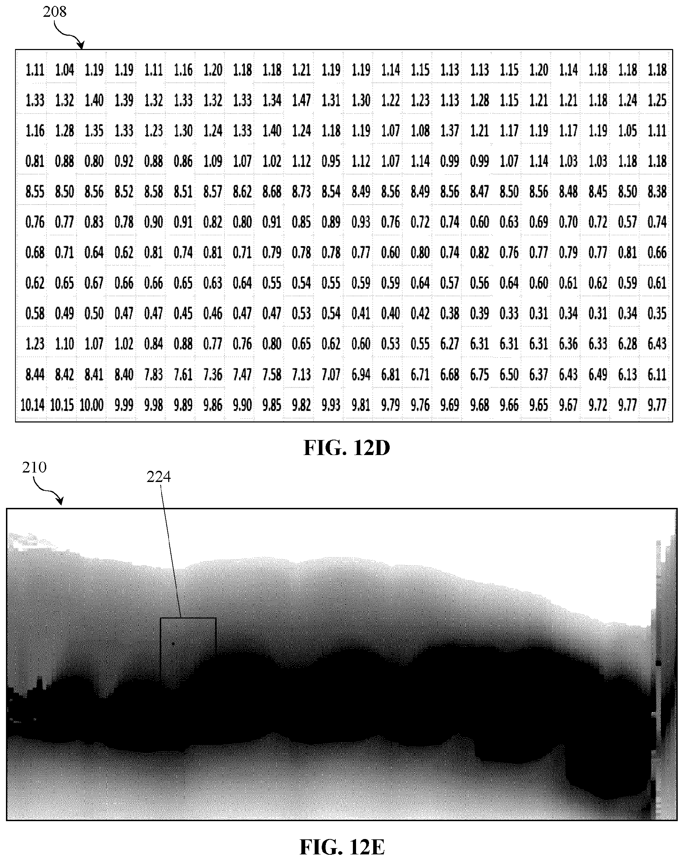

FIG. 12D shows an example of an output 2D matrix 208 of the Fourier neural operator for a dental object. The 2D matrix 208 may be generated from the radial encoding by the Fourier neural operator. FIG. 12D shows an example of an output 2D matrix 208 of the Fourier neural operator for the matrix portion of the tooth shown in FIG. 12D. Only the matrix values for the matrix portion shown in FIG. 12E is shown in 2D matrix 208 for ease of presentation. It should be understood that a full matrix may have many rows and columns, on the order of hundreds or thousands, to provide sufficient granularity for the entire dental object anatomy. FIG. 12E illustrates an example of a matrix visualization using black/white greyscale mapping to provide a visualization map 210. Each entry in the matrix may be used as a pixel value in a visualization mapping/image. In some embodiments, the number of rows is equal to the number of slicing planes generated by the indexed slicer, and the number of columns is equal to the number of ray lengths generated in each slice by the radial encoder. Each element of the matrix represents the distance from the slicing centroid to the cross-section boundary of that slice. The output of the Fourier neural operator is the 2D matrix can further be used in stacking applications described below. The 2D matrix can also be visualized to assist dentists and/or lab technicians for further analysis, as shown. The values of the Neural operator matrix may be mapped to rainbow colour, black and white shading, or any other colour mapping method. The minimum and maximum values of the 2D matrix may be identified and assigned to the selected colour map value extreme points to generate the colour map. The values in the 2D matrix are represented as shading shown in matrix portion 224. Again, a full matrix would correspond to the entire visualization mapping/image such that each conversion of a matrix entry to a colour/shading may be represented as a pixel in the visualization map 210.

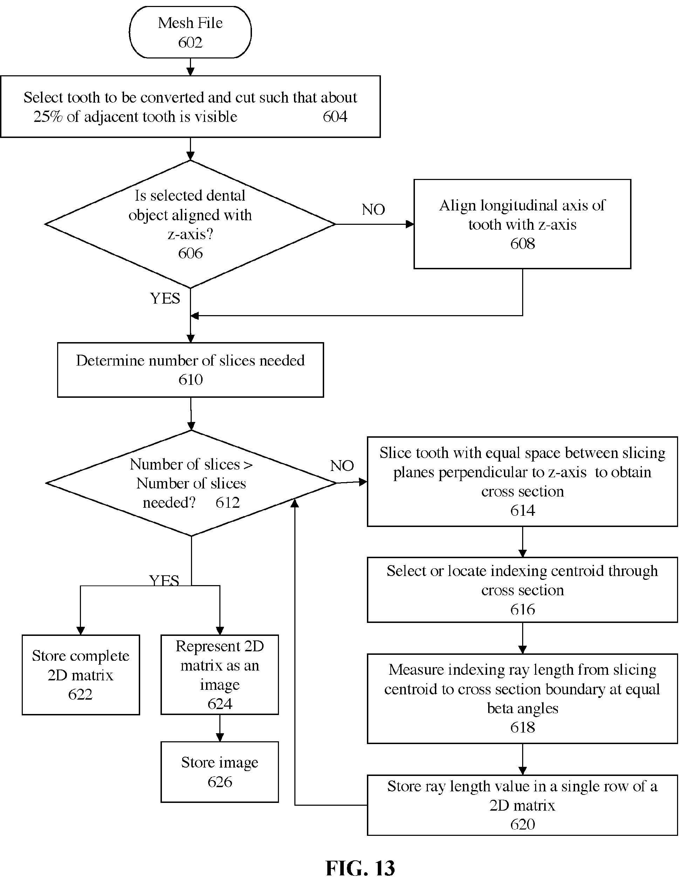

FIG. 13 illustrates, in a flowchart, another example of a method of generating a dental arch descriptor, in accordance with some embodiments. The method begins by taking a three-dimensional quadrant file 602 as an input. The quadrant shown in FIG. 12A is an example of an image of a quadrant file. The tooth or dental object to be converted is cut, or a boundary is applied, such that about 25% of adjacent teeth are visible or in the boundary box 604 to provide environmental context and structure for the tooth environment. In some embodiments, the system queries whether the z-axis is aligned with the anatomy's longitudinal axis 606. If it is not, then the anatomy's longitudinal axis is aligned with the z-axis 608. In some embodiments, the system automatically aligns the dental anatomy's longitudinal axis to the z-axis. For example, the system may generate a transformation matrix which moves the center of a bounding box of the input mesh or STL file to the origin. This aligns the dental anatomy's longitudinal axis aligned with the z-axis. The number of slices is then determined 610 to provide the data granularity desired, and then the system goes through an algorithmic loop which stops when the number of slices is greater than the number of slices needed 612. In the loop the tooth or post is sliced with equal spacing between each slice, where each slice is perpendicular to the z-axis to obtain a cross section 614, an indexing centroid for each cross-sectional slice is selected or located 616, the length of a plurality of indexing rays is measured from the indexing centroid to the cross-sectional boundary of the slice 618, and for each cross-section, each ray length is stored in a single row of a 2D matrix 620. By running this loop (steps 612 to 620) single row units for each slice can be obtained and stored in the matrix format one after another. This resulting 2D matrix may be stored 622 in a matrix database or dental descriptor database, and the 2D matrix may also be represented as an image 624 and the image can be stored 626. The method may also be performed for another tooth anatomy for example another targeted incisor, molar, or premolar three-dimensional teeth.

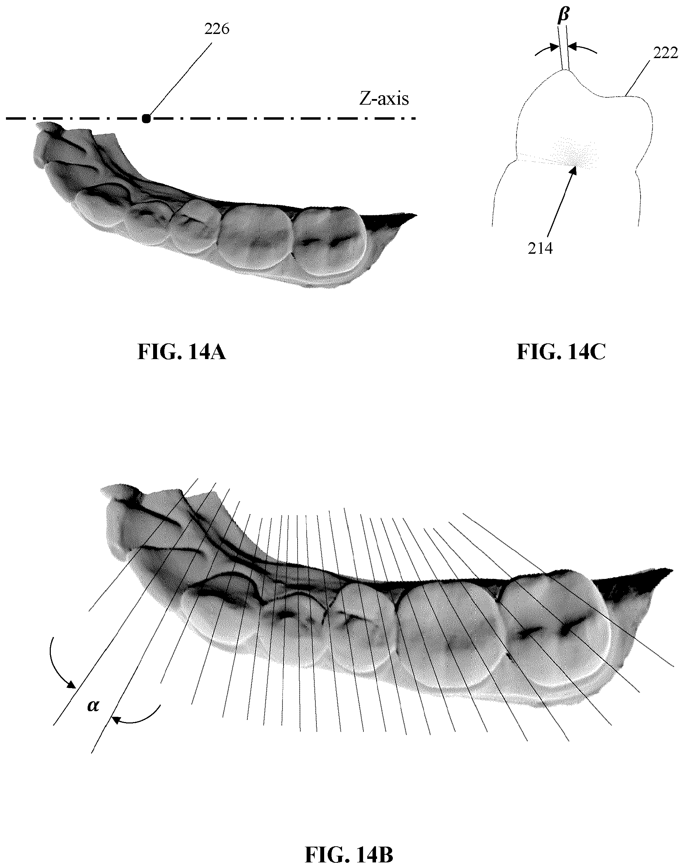

FIGs. 14A to 14C illustrate stages of obtaining slices of a dental arch, in accordance with some embodiments. FIG. 14A illustrates an example of the z-axis position for a quadrant in a dental arch. The alignment of the z-axis is performed to ensure the data is generated and stored uniformly. Next, arch centroid 226 is located or selected. Each mesh file has a bounding box, and the slicing centroid is preferably set as the volumetric centre of the bounding box. Alternatively, the slicing centroid can be assigned as the same as the volumetric centroid of the mesh file. In an arch description method, the arch centroid is preferably assigned as the volumetric centroid of the full dental arch. Selecting the slicing centroid in this way allows the comparison with similar full dental arches, which also have the same descriptor classification. FIG. 14B illustrates planar slices of the dental arch. The number of slices needed to ensure all features are covered is determined to provide the data density required for characterizing the dental arch. For example, if the number of slices is too few, then the space between slices may be large enough so that their cross-sections do not show an anomaly or feature of the anatomy that is desired for visualization and modeling. This step will determine the quality and size of the matrix and the resulting quality and density of the image or visualization map. If the number of slices is not greater than the number of slices needed then the arch is sliced radially at an approximately equal alpha angle, as a plane normal in a clockwise direction with the arch centroid as a plane origin to obtain a cross-section view for each slice. It should be noted that the slicing can be, for example, from right to left or from left, as long as all teeth are sliced consistently in the same manner or direction (i.e., always from left to right, or from right to left) in accordance with a dental file segmentation algorithm defining the slicing methodology and distances such that the resulting matrix values and array value mapping is comparable for similar dental anatomy in with the same descriptor classification. FIG. 14C shows a single slicing plane and dental object cross sectional boundary. An indexing centroid 214 is located for each cross-section, and the radial encoder divides the plane with a plurality of indexing ray. Each indexing ray has a radial length measured between the indexing centroid 214 and a cross-section boundary 222. The indexing rays can be assigned in a clockwise direction from a first indexing ray, or in an anti-clockwise direction. The beta angle between indexing rays can be approximately equal, or it can vary based on the area of the dental object that required the highest resolution. An output 2D matrix of the Fourier neural operator can be generated for the dental object. An output ray length value for each indexing ray the slicing plane is provided, and the values can be stored as a single row unit in a 2D matrix. The 2D matrix obtained can be stored in the matrix database. The matrix may also be stored in an image format. A visualization map may also be generated by representing the maximum and the minimum values in the matrix to chosen color extreme values and saving an image file corresponding to the tooth matrix. The image file format is not limited to any single file format and can be stored in a repository. The tooth database may also be linked with the image repository.

### Full Arch Descriptor:

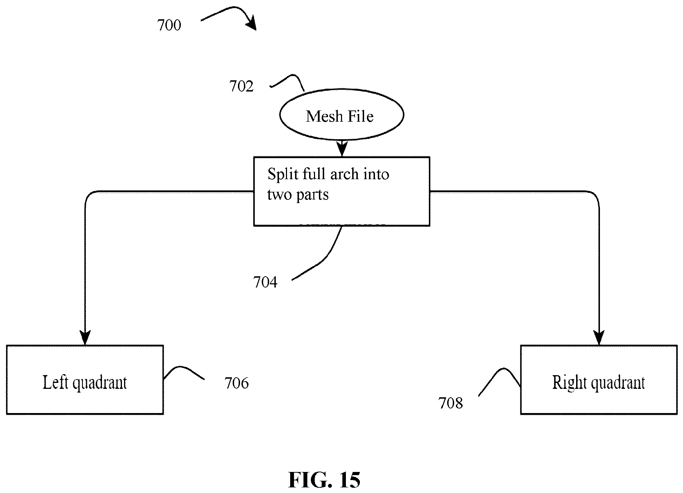

FIG. 15 illustrates, in a flowchart, an example of a method of generating a full arch descriptor 700, in accordance with some embodiments. This method of generating a full arch descriptor 700 employs an arch descriptor method as described above. In use the method starts with an STL or mesh file 702 of a full dental arch. The full arch is split into two parts 704, preferably around the central incisor. This gives two different quadrants, namely left quadrant 706 and right quadrant 708 as per where the quadrant is located. Each of the left quadrant 706 and right quadrant 708 can be treated as a separate independent quadrant, and arch descriptor method I or II may be applied to those to obtain one or more 2D matrix and/or visualization of the full arch or subset thereof, depending upon the method selected.

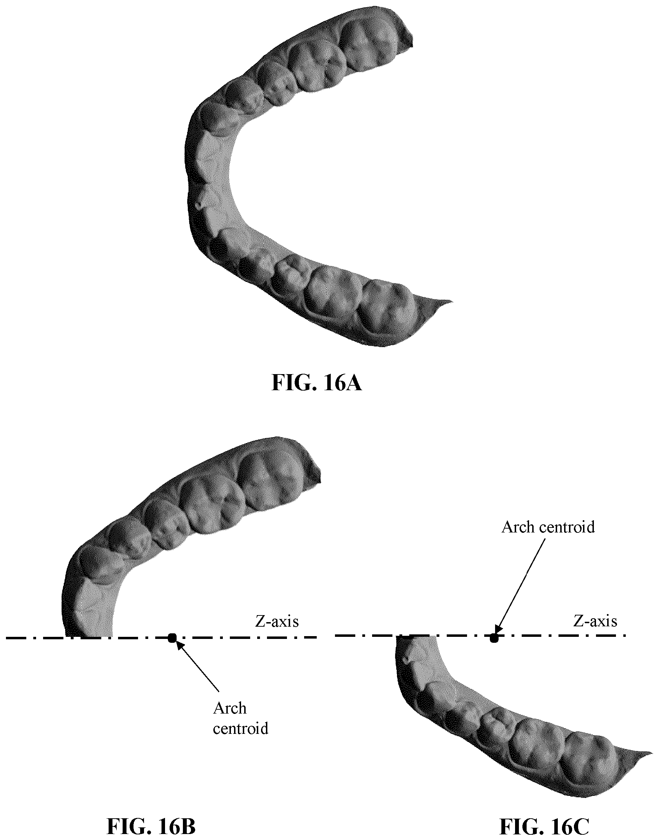

FIG. 16A illustrates an example of a full arch, in accordance with some embodiments. In this example, a lower arch of the patient is shown. It is understood that the same method can also be applied to the upper full arch. The method of generating a full arch descriptor splits a full arch STL or mesh file into two parts, preferably around the central incisor teeth. This gives two different quadrants, namely left quadrant and right quadrant as per where the quadrant is located. FIGs. 16B and 16C illustrate examples of half, of the full arch after splitting the full quadrant, in accordance with some embodiments. Each quadrant can be treated as a separate independent quadrant. Once collected, the 2D matrix describing each dental object processed is added to a descriptor database. Using matching algorithms, machine learning, and artificial intelligence, the 2D matrix representation of dental objects can be classified and matched to other dental objects in the descriptor database. By assigning descriptor classes to each 2D matrix data file, each 2D matrix can be passed through a neural network and a model is created for dental objects in the same descriptor class using the 2D matrix descriptions of the 3D dental object for matching. Using this method of algorithmic matching, similar pattern prediction can be accomplished, with classification of similar groups of 2D matrixes into related descriptor classes. By creating descriptor classes for the 2D matrixes in the descriptor database new dental features can be identified and patterns can be use in anomaly detection, and prosthetic design. For example, identification of patterns in a descriptor class which suggest disease or degradation will help dental professionals to identify problems more easily and earlier. In one example, gum recession can be measured and monitored, and patterns of gum recession from gum descriptors around the same tooth or teeth in the descriptor database, either from the same patient or a different patient, can assist in providing early diagnosis of gum disease. Diagnosing gum disease early can also more quickly initiate treatment which can prevent or delay progression. All the aspects of those examples including a diagnostic method are excluded from the claimed invention.

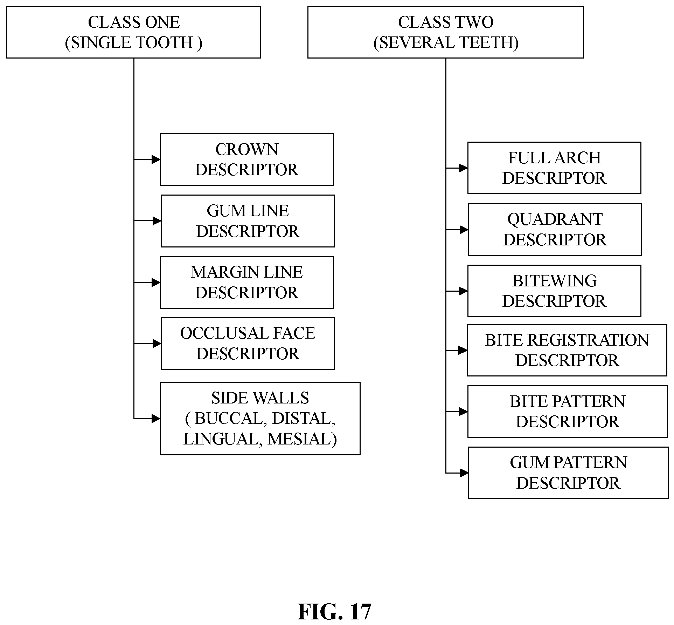

FIG. 17 illustrates dental descriptors for single and multiple dental features. In this schema, a class one dental feature is defined as a feature encapsulated by a single tooth, and a class two dental feature is defined as a feature that encompassed more than one tooth or complex dental object such as a gumline or dental arch. In the shown model there are ten descriptor subclasses. This is one example of descriptor classification, however it is understood that classification of dental features may be done in different ways. In a specific example, additional descriptor classes can be assigned by patient age, as the teeth and jaw grow and change from birth to adulthood. In the example shown, a class one set of descriptors describing single teeth features can include, for example, a crown descriptor, gumline descriptor, margin line descriptor, occlusal face descriptor, side walls descriptor. For single tooth descriptor classes, a single descriptor subclass can be created for each individual tooth, for example, by tooth number and/or tooth name. In particular, descriptor subclasses can be assigned for each of: upper right teeth 1-Central incisor, 2-Lateral incisor, 3-Canine/Cuspid, 4-First Premolar/1st Bicuspid, 5-Second Premolar/2nd Bicuspid, 6-First Molar, 7-Second Molar, 8-Third Molar/Wisdom tooth; upper left teeth 9-Central incisor, 10-Lateral incisor, 11-Canine/Cuspid, 12-First Premolar/1st Bicuspid, 13-Second Premolar/2nd Bicuspid, 14-First Molar, 15-Second Molar, 16-Third Molar/Wisdom tooth; lower left teeth 17-Central incisor, 18-Lateral incisor, 19-Canine/Cuspid, 20-First Premolar/1st Bicuspid, 21-Second Premolar/2nd Bicuspid, 22-First Molar, 23-Second Molar, 24-Third Molar/Wisdom tooth; and lower right teeth, 25-Central incisor, 26-Lateral incisor, 27-Canine/Cuspid, 28-First Premolar/1st Bicuspid, 29-Second Premolar/2nd Bicuspid, 30-First Molar, 31-Second Molar, 32-Third Molar/ Wisdom tooth. The present system can automatically classify each tooth using the 2D matrix, or the tooth can be classified manually using metadata accompanying the 2D matrix. Class two descriptors can work in a similar fashion, and can include descriptors which describe the features of several teeth, for example include full arch descriptor, quadrant descriptor, bitewing descriptor, bite registration descriptor, bite pattern descriptor, gum pattern descriptor. Each 2D matrix can be further classified by a descriptor subclass that specifies location of the dental object, such as upper right quadrant, upper left quadrant, lower right quadrant, lower left quadrant, bitewing location, occlusal surface and location, gumline and location, and dental arch.

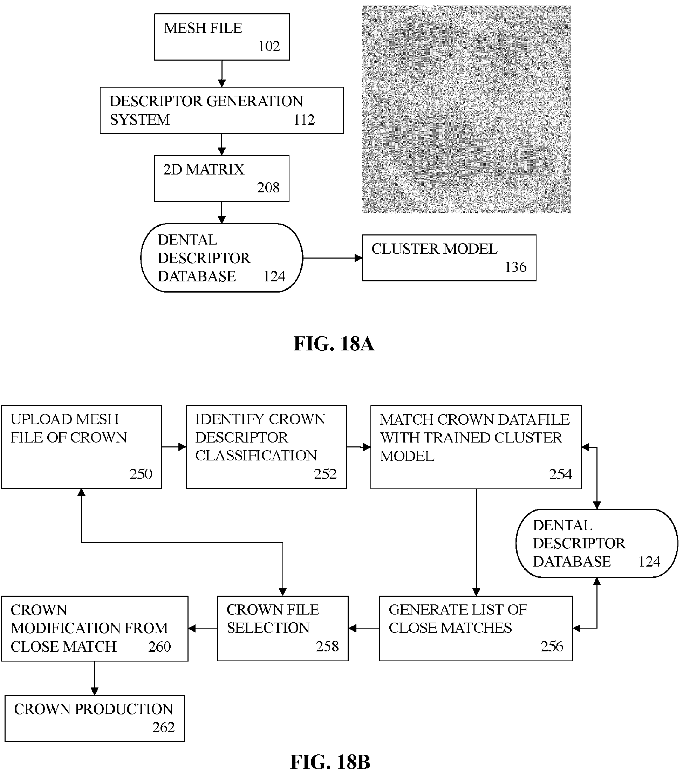

FIG. 18A describes one use case for class one descriptors. In this example, a class one descriptor, crown descriptor and the use case are shown demonstrating how the same can be used for clustering in an augmented intelligence using machine learning. A trained machine learning cluster model 136 is used for this workflow, which can be achieved by training a convolutional neural network by using a crown descriptor dataset in a dental descriptor database 124. In the clustering model, the method will create n different descriptor subclasses for the given dataset such that future input of 2D matrix 208 dental object data into the dental descriptor database 124 can be automatically directed it to its matching descriptor subclass. Once the training part is completed the trained model can be used in a production workflow. As shown, a mesh file 102 of a single tooth is uploaded and a descriptor subclass or tooth identification number is assigned to the mesh file, either by a dental technician or automatically by the system. To do this the mesh file 102 is processed by the descriptor generation system 112 which uses dental file segmentation to convert the mesh file 102 into a 2D matrix 208 representation of the mesh file of the single tooth. For generation of a crown, the tooth can be classified in a crown descriptor class, and the 2D matrix can be processed using a crown descriptor generator to general a crown descriptor datafile image specific to the tooth. The machine learning system having multiple classified dental object descriptors and at least one 2D matrix for each dental object descriptor can provide a training set for classification of teeth using 2D matrix file representations. Each generated 2D matrix describing a dental object can be stored in the dental descriptor database 124, which contains a plurality of dental object 2D matrix representations along with their descriptor class/subclass

FIG. 18B illustrates an example workflow for crown production in a computer-aided crown design process using a single tooth descriptor classification. The example shown is for a crown descriptor classification, however it is understood that any descriptor classification can be used in accordance with a similar method. In the example shown, once a crown has been imaged and a 2D matrix file has been generated for the crown, the dental pattern provided by the 2D matrix, which is representative of the crown, enables the present system to identify similar crowns within the same tooth/crown descriptor class, i.e. same tooth identification and similar crown pattern. The crown 3D mesh datafile is uploaded to a processor 250 along with the identification of the tooth/crown descriptor classification 252, in this case a crown descriptor and tooth location and/or tooth class identification. The 2D matrix datafile of the crown or image file visualization thereof, which in some embodiments includes the post and dental environment around the post such as adjacent teeth and gum structure, can then be processed using a trained machine learning cluster model to match the crown datafile with similar crown datafile using a trained cluster model 254. To match the 2D datafile, the descriptor classification and subclassification information will identify the correct classification, and then the trained model will match 2D datafiles of crowns with similar patterns in the dental descriptor database(s) 124 in the same descriptor class/subclass. The datafile matching is preferably done with a convolutional neural network with feature mapping of the 2D matrixes to identify close matches to the crown. The system then generates a list of close matches 256 for further processing. The system can also overlay two or more of the close matched to propose a mosaiced datafile for further crown processing. A dental professional can then select the crown file from the list of close matches 258 or the mosaic of close matches for optional further crown modification. In an example, different features can be selected from different closely matching 2D matrix files. For example, an occlusal surface feature from a first descriptor matrix may be mosaiced with a gumline margin feature from another matching 2D matrix file to provide a closer match which is a combination of the two that can be used as a base model for further customization of the crown. Modification to the selected crown or crown mosaic that is a close match can be done 260, and the final CAD file can be sent to a manufacturing system for crown manufacturing and production 262. In contrast, to create a crown using current technology, a dental technician generally starts with a generic crown model without features and adds dental features based on the tooth shape of the patient for which the crown is being made. This process is extremely time consuming and takes a lot of time for dental technicians, making it the slowest part of crown design and manufacture. The present process drastically speeds up crown design by providing the dental technician with a highly similar crown model to work from, for which only minor modification is required. Once the final crown is designed the dental technician can then send the crown datafile into production to create the crown.

### Array Mapping Method

Consistent value mapping describing of 3D objects using the same slicing and indexing algorithms and constraints, such as incremental alpha angle, beta angle, or slicing plane distance, across the same descriptor class and subclass can be used effectively for stacking multiple arrays describing 3D patterns of objects. The concept here is the ability to map data value locations of each feature on the corresponding array of all objects of the same class. In a single tooth descriptor class, a fabricated crown descriptor can be the output of a computer aided design process as presently described. In an example, a fabricated crown can be described using three arrays sharing the same value mapping, where each array describes a different feature of the crown, specifically the crown shape, crown abutment with gum, and the crown thickness. Neural operators allow the visualization of relevant dental features, and features can be clustered on a large database efficiently using a machine learning method. In this example, the relevant dental features crown shape, crown abutment with gum, and the crown thickness, can be integrated into the computer-assisted design process. By combining a set of desirable features, a fabricated crown design can be then designed efficiently by tweaking targeted values mapped on arrays for seamless integration of each 3D feature into one design file.

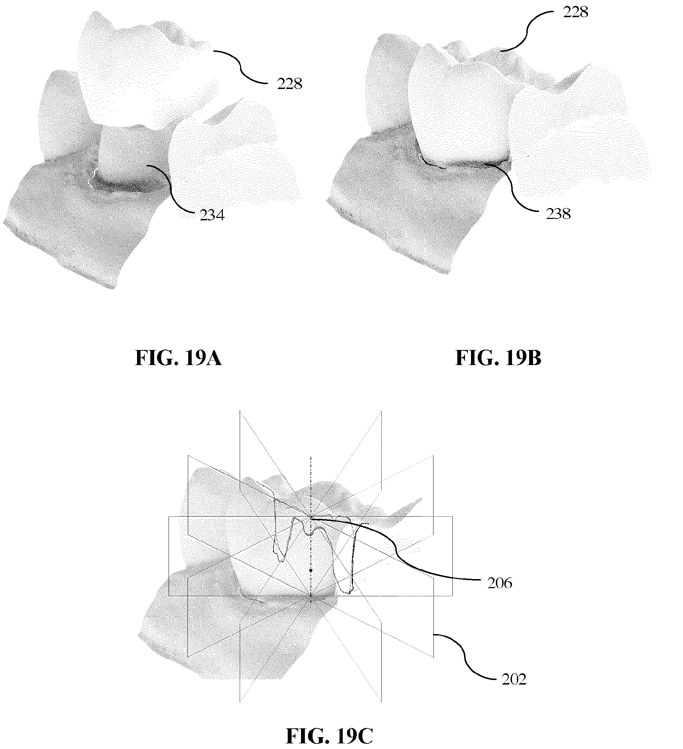

FIG. 19A illustrates an array mapping procedure for crown and crown post fitting. The term 'crown' as referred to herein refers to the fabricated dental crown which caps a post, where the post is the remainder of a tooth that requires capping. Fabricated dental crown 228 is designed to fit securely on post 234 such that it abuts the gum at a gumline abutment, also referred to herein as a margin. The position of the crown relative to the abutment can be visualized by using 2D matrixes to match or dock the crown 228 to the post 234. The relative angle and docking position of the crown 228 relative to the post 234 and the gumline are shown, in addition to the neighbouring teeth adjacent the post.

FIG. 19B illustrates a crown environment fitting with a fitted crown on a crown post. The design of dental crowns is a highly technical procedure, and creating a good crown can take a lot of technician time. Even with a well-designed crown, the area of the crown around the gumline often requires at least small adjustment to ensure that the crown shape has a good fit. This illustration shows how the fabricated dental crown 228 is mounted at the gumline at abutment or margin 238. This intersection between the crown 228 and gumline is also referred to as the margin, which is where the edge of the crown 228 matches with or mates with the gumline. The rotation and docking of the crown 228 into the post are important considerations for ensuring proper fit of the crown at the gumline. Two or more matrixes or arrays can be created which describe the fabricated crown 228 and the gumline abutment margin 238 by using the encoding method described herein. A first 2D matrix descriptor of the crown and second 2D matrix descriptor of the post can be compared during crown design to ensure that the crown fits properly onto the post. Additionally, a 2D matrix descriptor of the gumline around the post can assist in matching the crown with the margin 238 along the gum line to ensure the proper rotation angle and fitting of the crown into the margin 238. One or more further 2D matrix or part thereof can be used to match the occlusal surface of the crown with another pre-designed crown to design the top of the crown such that it is in line with the occlusal surfaces of neighbouring teeth. The margin 238 can be described in the first 2D matrix of the crown and second 2D matrix descriptor of the post and the crown design can be adjusted mathematically such that it matches the margin at the gumline around the post. The 2D matrix of the post and the 2D matrix of the crown are strongly related and important to match, and each point at the surface of the abutment margin 238 must be related and matching between the two matrixes to enable good crown fit. Additionally, the inside of the first 2D matrix descriptor of the crown and the exterior of the post in the second 2D matrix descriptor of the post and post environment must be matching in order to achieve a snug and well-fitting crown. Accordingly, the matrix of the first 2D matrix descriptor of the crown and second 2D matrix descriptor of the post must be matching and related in a volume and 3D structure such that the features have matching shape on the interior as well as at the margin of the crown. Mathematically, each point of the crown matrix and the abutment of the tooth environment matrix should be matching in order to obtain a good crown fit.

FIG. 19C illustrates a piece of an array mapping procedure for crown fitting. The position of crown slicing centroid 206 which is at the z-axis to the crown and crown post and crown radial slicing planes 202 along with the alpha angle between each slicing plane are chosen strategically so as to generate each 2D descriptor matrix with sufficient details to accurately model the crown in a 2D matrix. At least two matrixes are used for crown design, specifically a first 2D matrix of the crown and a second 2D matrix descriptor of the post and teeth environment around the post. The two matrixes can then be processed together for radial slicing such that both matrixes use the same slicing centroid in order that the matrixes value mapping are axially aligned. In one example, if the same centroids are used in both the slicing and radial encoding methods, the resulting set of 2D matrixes as modified by the neural operator provide the ability to map the data systematically across a multitude of 2D matrix datafiles and thereby enable ease in searching in the matrix database for matching dental objects, crowns, and matching features thereof. In particular, sharing a common slicing centroid in the process of generating the two matrixes ensures value mapping matching between the resulting 2D matrixes. By standardizing the dental file segmentation algorithm, at least across descriptor classifications, the present method can more easily find similar matches for dental crown size, shape, and thickness in the 2D matrix database. As shown crown slicing centroid 206 and crown radial slicing planes 202 are shown which are used for the index slicing and radial encoding methods, respectively. In a future crown design with an existing 2D matrix of the post for which the crown is being designed, aligning the new crown to the same slicing centroid further enables accurate crown matching and design in the post environment.

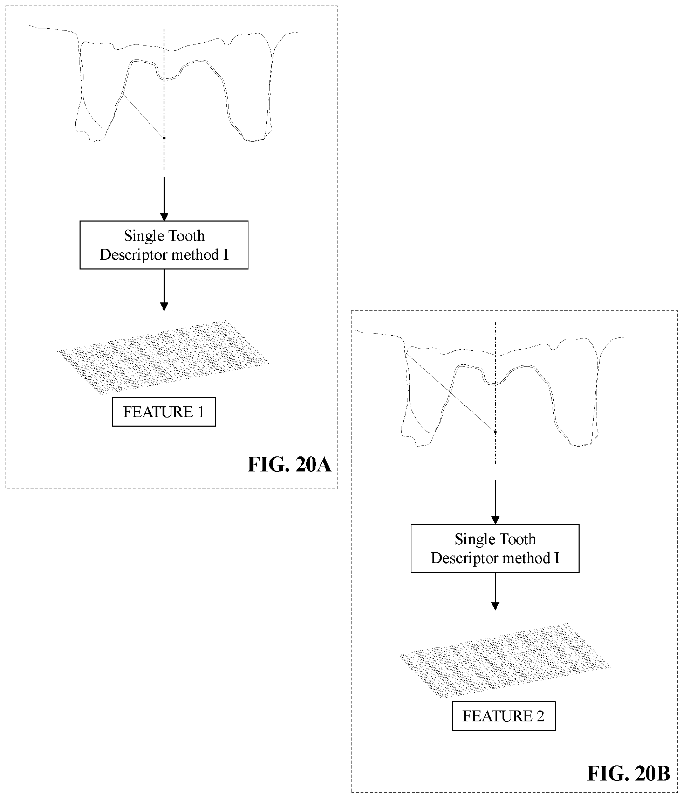

FIG. 20A is an illustration of a crown mounted onto a post showing a cross section of the abutment surface between the inside of the crown and the outside of the post. In this case, the abutment surface encompasses the 3D surface locations where the inside of the crown meets the post surface as well as the margin of the crown around the gumline. The abutment surface can also be thought of as the surface inside the crown up to the crown margin where the crown margin abuts the gumline. The crown post, or simply post, can be thought of as the remaining part of the tooth on which a fabricated crown can be attached. An abutment matrix is shown which describes the abutment surface and the margin at the gumline, identified as FEATURE 1. There are a variety of features that can be used to describe the crown and environment that the crown occupies, including but not limited to an abutment surface to a post, margin at the gumline, occlusal surface, crown thickness, groove and pit shape and location, abutment surface or space between neighbouring teeth, and other features. In designing a crown each feature can be represented by a different 2D matrix or different part of the 2D matrix.

FIG. 20B is an illustration of a crown mounted onto a post showing a cross section of the abutment surface between the outside of the crown and the distal and mesial abutments to neighbouring teeth as well as the crown thickness at various places in the crown. The same descriptor can be used to describe the crown 2D matrix, with differences in which distance the radial encoder measures. In this case, FEATURE 2 describes the crown thickness and exterior crown surface. The same indexing centroid can be used to represent FEATURE 2 as the crown and post abutment described in FEATURE 1, but now each indexing ray will be extended to the outer surface of the crown as shown in the sample slice. Performing two separate radial encoding steps enables different features of the crown and tooth to be represented.

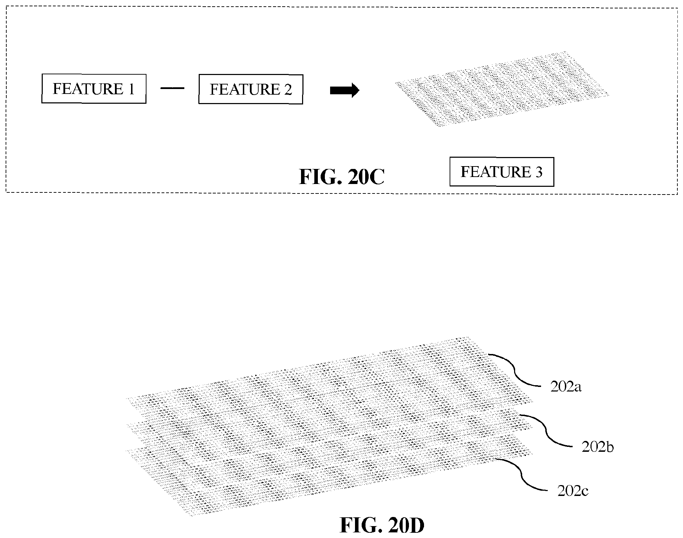

FIG. 20C is a visual representation of a combination of feature matrixes to provide a fulsome value mapping of a crown. By combining a 2D matrix representing the abutment surface between the inside of the crown and the outside of the post as shown in FEATURE 1 with a 2D matrix of the abutment surface between the outside of the crown and the distal and mesial abutments to neighbouring teeth as shown in FIGURE 2, where each feature has been value mapped with a coaxial radial encoding axis, a difference in the 2D matrixes can provide the crown thickness as FEATURE 3, which is another value mapping 2D matrix representing another feature of the crown in its dental environment.

FIG. 20D illustrates an example of a matrix stacking of a plurality of 2D matrixes, with each matrix providing a value mapping of a different feature of the crown in its dental environment. The 2D matrix stacking can enable extraction of single features of the crown, for example, only the margin line. The occlusal surface can also be represented by a matrix stacking of two or more 2D matrixes with a coaxial centroid. From a plurality of stored 2D matrixes 202a, 202b, 202c of a plurality of crowns and teeth a unique crown can be assemble in parts that match with multiple different features of multiple different crowns/teeth. In an example, the post abutment surface, occlusal surface, and distal/mesial abutment surfaces etc. can be taken from multiple 2D matrixes 202a, 202b, 202c in the database to create a stacked matrix in order to more closely arrive at an ideal starting position or match for the new crown, where the match is comprised of a plurality of stacked matrixes. In this way the system can match a dental pattern from a plurality of teeth and crown examples to arrive at a good base match which is a mosaic of a plurality of crown examples. The mosaic of matches can then be used as a starting design for a new crown, with the starting design taking into consideration both the mesh file of the patient's teeth and one or more example close matches of pre-created crown designed. The resulting base match will be very close to a good match, reducing the effort required of the prosthetic designer to produce an optimal design for the patient.

### Array Value Mapping for Descriptors having Multiple Teeth

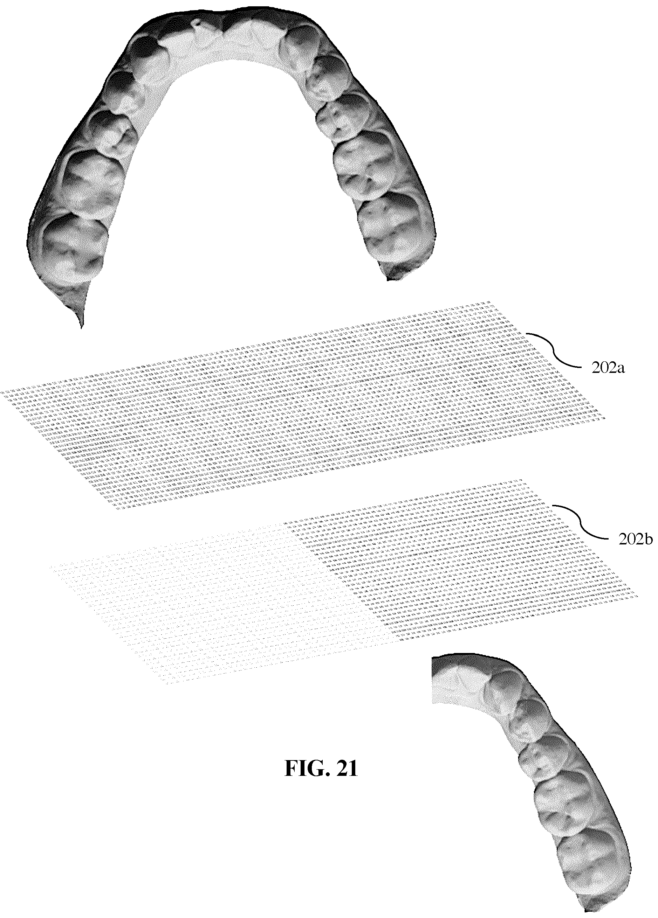

FIG. 21 illustrates an array value mapping method for a dental arch with a 2D descriptor matrix 202a. The mapping of a 2D descriptor matrix 202a that matches and describes the full dental arch comprises descriptors for multiple teeth in the dental arch, and may be used for an arch, bitewing, or dental quadrant. Two mesh files are shown, one 2D matrix 202a of the full dental arch (top) and a second 2D matrix 202b of half of the dental arch, or quadrant. In this example, the second 2D matrix 202b of half of the dental arch is simply half of the value mapped 2D matrix 202a of the full dental arch. By encoding the 2D matrixes in this way fractions of features, for example structural or abutment features, can be extracted from the full 2D matrix of a full dental arch. The resulting value mapped descriptor file, or 2D matrix 202b of the quadrant descriptor, is half the size of full arch descriptor 2D matrix 202a. If the value mapped data is organized in this way the quadrant matrix can easily be extracted from the full arch matrix. In the same way, the 2D matrix value mapping of a single tooth can be similarly extracted from the 2D matrix of a dental object, such as the dental arch or quadrant, that contains the single tooth structure. In this way the value mapping and resulting 2D matrix descriptor is related to and is a mathematically representation of the actual 3D shape of the dental object.

The of the invention is defined by the following claims.

## Claims

1. A computer-implemented method for dental file segmentation comprising:
receiving (402) a 3D mesh file representation of a 3D dental object;
aligning (404) a longitudinal axis of the 3D mesh file with a z-axis;
assigning (406) a slicing centroid to the 3D mesh file;
slicing (408) the 3D mesh file into a plurality of cross-sectional slices; and
for each 2D cross-sectional slice:
identifying (416) an indexing centroid;
measuring (418) a length of each of a plurality of indexing rays to provide a plurality of lengths, each of the plurality of indexing rays extending from the indexing centroid to a cross-sectional boundary of the cross-sectional slice; and
storing (420) the plurality of lengths in a row in a 2D matrix to create a 2D descriptor matrix of the 3D mesh file.

2. The method of claim 1, further comprising classifying the dental object with a dental descriptor.

3. The method of claim 2, wherein the dental descriptor comprises one or more of dental object location, tooth identification, quadrant location, dental arch, bitewing, occlusal surface, and gumline.

4. The method of any of claims 1-3, wherein the plurality of cross-sectional slices are separated by a selected alpha angle and/or wherein the plurality of indexing rays in each 2D cross-sectional slice are separated by a selected beta angle.

5. The method of any of claims 1-4, further comprising matching the 2D matrix to one or more similar 2D matrix in a dental object database.

6. The method of claim 5, further comprising using the selected one or more similar 2D matrix to design a dental prosthetic, the method preferably further comprising manufacturing the dental prosthetic.

7. The method of any of claims 1-6, further comprising rendering the 2D matrix as a visualization map image.

8. The method of claim 7, wherein the visualization map is rendered such that each entry in the descriptor matrix is replaced with a corresponding color.

9. The method of any of claims 1-8, wherein slicing the 3D mesh file comprises radially slicing the dental object through the slicing centroid.

10. The method of any of claims 1-9, wherein slicing the 3D mesh file comprises longitudinally slicing the dental object into substantially parallel slices.

11. The method of any of claims 1-10, wherein the dental object is one or more of a single tooth, multiple teeth, two or more adjacent teeth, full dental arch, half dental arch, dental quadrant, bitewing, and gumline.

12. A computer implemented system (100) for dental file segmentation comprising:
a dental object descriptor generating system (112) comprising:
an indexed slicer (104) for receiving a 3D mesh file of a dental object and slicing the mesh file into a plurality of slices, each slice comprising a cross-sectional boundary of the dental object;
a radial encoder (106) assigning an indexing centroid and measuring a plurality of rays from the indexing centroid to the cross-sectional boundary;
a Fourier neural operator unit (108) to transform the plurality of ray lengths into a 2D descriptor matrix of the dental object; and
a descriptor database (124) comprising a plurality of 2D descriptor matrixes for a plurality of dental objects, each 2D matrix having a descriptor classification.

13. The system of claim 12, further comprising a cluster model for matching two or more of the plurality of 2D descriptor matrixes in the dental object database.

14. The system of claim 12 or 13, further comprising a computer-aided design system for designing a dental prosthetic based on the 2D descriptor matrix.

15. The system of any of claims 12-14, further comprising a dental scanner (132).

## Patentansprüche

1. Computerimplementiertes Verfahren zur Segmentierung von zahnmedizinischen Dateien, umfassend:
Empfangen (402) einer 3D-Mesh-Datei-Darstellung eines zahnmedizinischen 3D-Gegenstands;
Ausrichten (404) einer Längsachse der 3D-Mesh-Datei auf eine z-Achse;
Zuweisen (406) eines Slicing-Schwerpunkts zur 3D-Mesh-Datei;
Slicing (408) der 3D-Mesh-Datei in eine Vielzahl von Querschnitts-Slices; und
für jeden 2D-Querschnitts-Slice:
Identifizieren (416) eines Indexierungs-Schwerpunkts;
Messen (418) einer Länge jedes einer Vielzahl von Indexierungsstrahlen, um eine Vielzahl von Längen bereitzustellen, wobei sich jeder der Vielzahl von Indexierungsstrahlen vom Indexierungs-Schwerpunkt zu einer Querschnittsgrenze des Querschnitts-Slice erstreckt; und
Speichern (420) der Vielzahl von Längen in einer Zeile in einer 2D-Matrix, um eine 2D-Deskriptormatrix der 3D-Mesh-Datei zu erstellen.

2. Verfahren nach Anspruch 1, das ferner das Klassifizieren des zahnmedizinischen Gegenstands mit einem zahnmedizinischen Deskriptor umfasst.

3. Verfahren nach Anspruch 2, wobei der zahnmedizinische Deskriptor eine oder mehrere von Lage des zahnmedizinischen Gegenstands, Zahnidentifikation, Quadrantenlage, Zahnbogen, Bissflügel, Okklusionsfläche und Zahnfleischrand umfasst.

4. Verfahren nach einem der Ansprüche 1-3, wobei die Vielzahl von Querschnitts-Slices durch einen ausgewählten Alpha-Winkel voneinander getrennt sind und/oder wobei die Vielzahl von Indizierungsstrahlen in jedem 2D-Querschnitts-Slice durch einen ausgewählten Beta-Winkel voneinander getrennt sind.

5. Verfahren nach einem der Ansprüche 1-4, das ferner das Abgleichen der 2D-Matrix mit einer oder mehreren ähnlichen 2D-Matrizen in einer Datenbank für zahnmedizinische Gegenstände umfasst.

6. Verfahren nach Anspruch 5, das ferner die Verwendung der ausgewählten einen oder mehrerer ähnlicher 2D-Matrizen zur Konstruktion eines Zahnersatzes umfasst, wobei das Verfahren vorzugsweise ferner die Herstellung des Zahnersatzes umfasst.

7. Verfahren nach einem der Ansprüche 1-6, das ferner das Rendern der 2D-Matrix als Visualisierungskartenbild umfasst.

8. Verfahren nach Anspruch 7, wobei die Visualisierungskarte so dargestellt wird, dass jeder Eintrag in der Deskriptormatrix durch eine entsprechende Farbe ersetzt wird.

9. Verfahren nach einem der Ansprüche 1-8, wobei das Slicing der 3D-Mesh-Datei das radiale Slicing des zahnmedizinischen Gegenstands durch den Slicing-Schwerpunkt umfasst.

10. Verfahren nach einem der Ansprüche 1-9, wobei das Slicing der 3D-Mesh-Datei das Längs-Slicing des zahnmedizinischen Gegenstands in im Wesentlichen parallele Slices umfasst.

11. Verfahren nach einem der Ansprüche 1-10, wobei der zahnmedizinische Gegenstand eines oder mehrere von einem einzelnen Zahn, mehreren Zähnen, zwei oder mehr benachbarten Zähnen, einem vollständigen Zahnbogen, einem halben Zahnbogen, einem Zahnquadrant, einem Bissflügel und dem Zahnfleischrand ist.

12. Computerimplementiertes System (100) zur Segmentierung von zahnmedizinischen Dateien, umfassend:
ein System (112) zur Erzeugung eines Deskriptors für einen zahnmedizinischen Gegenstand, umfassend:
einen indexierten Slicer (104) zum Empfangen einer 3D-Mesh-Datei eines zahnmedizinischen Gegenstands und zum Slicing der Mesh-Datei in eine Vielzahl von Slices, wobei jeder Slice eine Querschnittsgrenze des zahnmedizinischen Gegenstands umfasst;
einen Radial-Encoder (106), der einen Indexierungs-Schwerpunkt zuweist und eine Vielzahl von Strahlen vom Indexierungs-Schwerpunkt zur Querschnittsgrenze misst;
eine Fourier-Neuraloperatoreinheit (108) zur Transformation der Vielzahl von Strahllängen in eine 2D-Deskriptormatrix des zahnmedizinischen Gegenstands; und
eine Deskriptordatenbank (124), die eine Vielzahl von 2D-Deskriptormatrizen für eine Vielzahl von zahnmedizinischen Gegenständen umfasst, wobei jede 2D-Matrix eine Deskriptorklassifizierung aufweist.

13. System nach Anspruch 12, das ferner ein Clustermodell zum Abgleichen von zwei oder mehr der Vielzahl von 2D-Deskriptormatrizen in der Datenbank zahnmedizinischer Gegenstände umfasst.

14. System nach Anspruch 12 oder 13, das ferner ein computergestütztes Konstruktionssystem zum Entwerfen eines Zahnersatzes auf der Grundlage der 2D-Deskriptormatrix umfasst.

15. System nach einem der Ansprüche 12-14, das ferner einen zahnmedizinischen Scanner (132) umfasst.

## Revendications

1. Procédé mis en œuvre par ordinateur pour la segmentation de fichier dentaire comprenant :
la réception (402) d'une représentation de fichier de maillage 3D d'un objet dentaire 3D ;
l'alignement (404) d'un axe longitudinal du fichier de maillage 3D avec un axe z ;
l'attribution (406) d'un centroïde de segmentation au fichier de maillage 3D ;
la segmentation (408) du fichier de maillage 3D en une pluralité de tranches transversales ; et
pour chaque tranche transversale 2D :
l'identification (416) d'un centroïde d'indexation ;
la mesure (418) d'une longueur de chacun d'une pluralité de rayons d'indexation pour fournir une pluralité de longueurs, chacun de la pluralité de rayons d'indexation s'étendant du centroïde d'indexation jusqu'à une limite de section transversale de la tranche transversale ; et
le stockage (420) de la pluralité de longueurs dans une ligne d'une matrice 2D pour créer une matrice de descripteurs 2D du fichier de maillage 3D.

2. Procédé selon la revendication 1, comprenant en outre la classification de l'objet dentaire à l'aide d'un descripteur dentaire.

3. Procédé selon la revendication 2, dans lequel le descripteur dentaire comprend un ou plusieurs éléments parmi l'emplacement de l'objet dentaire, l'identification de la dent, l'emplacement par quadrant, l'arcade dentaire, la prise de vue interproximale, la surface occlusale et la ligne gingivale.

4. Procédé selon l'une quelconque des revendications 1 à 3, dans lequel la pluralité de tranches transversales sont séparées par un angle alpha sélectionné et/ou dans lequel la pluralité de rayons d'indexation dans chaque tranche transversale 2D sont séparés par un angle bêta sélectionné.

5. Procédé selon l'une quelconque des revendications 1 à 4, comprenant en outre la mise en correspondance de la matrice 2D avec une ou plusieurs matrices 2D similaires dans une base de données d'objets dentaires.

6. Procédé selon la revendication 5, comprenant en outre l'utilisation des une ou plusieurs matrices 2D similaires sélectionnées pour concevoir une prothèse dentaire, le procédé comprenant de préférence, en outre, la fabrication de la prothèse dentaire.

7. Procédé selon l'une quelconque des revendications 1 à 6, comprenant en outre le rendu de la matrice 2D sous la forme d'une image de carte de visualisation.

8. Procédé selon la revendication 7, dans lequel la carte de visualisation est rendue de telle sorte que chaque entrée dans la matrice de descripteurs est remplacée par une couleur correspondante.

9. Procédé selon l'une quelconque des revendications 1 à 8, dans lequel la segmentation du fichier de maillage 3D comprend la segmentation radiale de l'objet dentaire en passant par le centroïde de segmentation.

10. Procédé selon l'une quelconque des revendications 1 à 9, dans lequel la segmentation du fichier de maillage 3D comprend la segmentation longitudinale de l'objet dentaire en tranches sensiblement parallèles.

11. Procédé selon l'une quelconque des revendications 1 à 10, dans lequel l'objet dentaire est un ou plusieurs éléments parmi une dent unique, plusieurs dents, deux dents adjacentes ou plus, une arcade dentaire complète, une demi-arcade dentaire, un quadrant dentaire, une prise de vue interproximale et une ligne gingivale.

12. Système mis en œuvre par ordinateur (100) pour la segmentation de fichier dentaire comprenant :
un système de génération de descripteurs d'objet dentaire (112) comprenant :
un trancheur indexé (104) destiné à recevoir un fichier de maillage 3D d'un objet dentaire et à segmenter le fichier de maillage en une pluralité de tranches, chaque tranche comprenant une limite de section transversale de l'objet dentaire ;
un codeur radial (106) attribuant un centroïde d'indexation et mesurant une pluralité de rayons du centroïde d'indexation jusqu'à la limite de section transversale ;
une unité d'opérateur neuronal de Fourier (108) destinée à transformer la pluralité de longueurs de rayon en une matrice de descripteurs 2D de l'objet dentaire ; et
une base de données de descripteurs (124) comprenant une pluralité de matrices de descripteurs 2D pour une pluralité d'objets dentaires, chaque matrice 2D ayant une classification de descripteurs.

13. Système selon la revendication 12, comprenant en outre un modèle de regroupement pour faire correspondre deux matrices ou plus de la pluralité de matrices de descripteurs 2D dans la base de données d'objets dentaires.

14. Système selon la revendication 12 ou 13, comprenant en outre un système de conception assistée par ordinateur pour concevoir une prothèse dentaire sur la base de la matrice de descripteurs 2D.

15. Système selon l'une quelconque des revendications 12 à 14, comprenant en outre un scanner dentaire (132).
